# EUROPEAN PATENT APPLICATION

(11) **EP 4 156 194 A1**
(43) Date of publication of application: **29.03.2023**
(21) Application number: 22190841.1
(22) Date of filing: 14.01.2015
(51) Int. Cl.: G16B 20/00, G16B 50/10

(54) **METHODS AND SYSTEMS FOR GENOME ANALYSIS**

(30) Priority: 14.01.2014 US 201461927459 P
(62) Divisional of application: 15737458.8
(71) Applicant: Fabric Genomics, Inc., Oakland, CA 94612 (US); University of Utah, Salt Lake City, UT 84108 (US)
(72) Inventor: SINGLETON, Marc, Salt Lake City, 84116 (US); REESE, Martin, Oakland, 94602 (US); EILBECK, Karen, Salt Lake City, 84108 (US); YANDELL, Mark, Salt Lake City, 84108 (US)
(74) Representative: Avidity IP

(57) **Abstract**

The present disclosure provides methods and systems for prioritizing phenotype-causing genomic variants. The methods include using variant prioritization analyses and in combination with biomedical ontologies using a sophisticated re-ranking methodology to re-rank these variants based on phenotype information. The methods can be useful in any genomics study and diagnostics; for example, rare and common disease gene discovery, tumor growth mutation detection, drug responder studies, metabolic studies, personalized medicine, agricultural analysis, and centennial analysis.

## Description

### CROSS-REFERENCE

This application claims the benefit of U.S. Provisional Patent Application Serial No. 61/927,459, filed January 14, 2014, which is incorporated by reference herein in its entirety.

### STATEMENT AS TO FEDERALLY SPONSORED RESEARCH

This invention was made with government support under grant numbers R44HG3667, R43LM10874, R43HG6579 and R44HG6579. The government has certain rights in the invention.

### BACKGROUND

Manual analysis of personal genome sequences is a massive, labor-intensive task. Although much progress is being made in deoxyribonucleic nucleic acid (DNA) sequence read alignment and variant calling, little methods yet exist for the automated analysis of personal genome sequences. Indeed, the ability to automatically annotate variants, to combine data from multiple projects, and to recover subsets of annotated variants for diverse downstream analyses is becoming a critical analysis bottleneck.

Researchers are now faced with multiple whole genome sequences, each of which has been estimated to contain around 4 million variants. This creates a need to efficiently prioritize variants so as to efficiently and effectively allocate resources for further downstream analysis, such as external sequence validation, additional biochemical validation experiments, further target validation such as that performed routinely in a typical Biotech/Pharma discovery effort, or in general additional variant validation. Such relevant variants are also called phenotype-causing genetic variants.

### SUMMARY

In light of at least some of the limitations of current methods and systems, recognized herein is the need for improved methods and systems for genomic analysis.

The present disclosure provides methods and systems that can automatically annotate variants, combine data from multiple projects, and recover subsets of annotated variants for diverse downstream analyses. Methods and systems provided herein can efficiently prioritize variants so as to efficiently and effectively allocate resources for further downstream analysis, such as external sequence validation, additional biochemical validation experiments, further target validation, and additional variant validation.

In an aspect, the present disclosure provides a computer system for identifying phenotype-causing genetic variants, comprising computer memory having a plurality of phenotype causing genes or genetic variants; and a computer processor coupled to the computer memory and the database, wherein the computer processor is programmed to (i) identify a first set of phenotype causing genes or genetic variants, which first set of phenotype causing genes or genetic variants is among the plurality of phenotype causing genes or genetic variants in the computer memory; (ii) prioritize the first set of phenotype causing genes or genetic variants based on knowledge resident in one or more biomedical ontologies in a database; (iii) automatically identify and report a second set of phenotype causing genes or genetic variants, wherein a priority ranking associated with genes or genetic variants in the second set of genes and genetic variants is improved compared to a priority ranking associated with the first set of phenotype causing genes or genetic variants.

In some embodiments, the database is separate from the computer system. In some embodiments, the system further comprises a communication interface for obtaining genetic information of a subject. In some embodiments, the computer processor is further programmed to use the second set of phenotype causing genes or genetic variants to analyze the genetic information of the subject to identify a phenotype or disease condition in the subject. In some embodiments, the computer processor is further programmed to generate a report that indicates the phenotype or disease condition in the subject.

In some embodiments, the computer processor is further programmed to generate a report includes a diagnosis of a disease in the subject and/or recommends a therapeutic intervention for the subject. In some embodiments, the report is provided for display on a user interface on an electronic display.

In some embodiments, the computer processor is further programmed to provide the second set of phenotype causing genes or genetic variants on a user interface.

In another aspect, the present disclosure provides a method for identifying phenotype-causing genetic variants, comprising (a) providing a computer processor coupled to computer memory that includes a plurality of phenotype causing genes or genetic variants, wherein the computer processor is programmed to identify and prioritize sets of phenotype causing genes or genetic variants among the plurality of phenotype causing genes or genetic variants; (b) using the computer processor to identify a first set of phenotype causing genes or genetic variants, which first set of phenotype causing genes or genetic variants is among the plurality of phenotype causing genes or genetic variants in the computer memory; (c) prioritizing the first set of phenotype causing genes or genetic variants based on knowledge resident in one or more biomedical ontologies; and (d) automatically identifying and reporting on a user interface a second set of phenotype causing genes or genetic variants, wherein a priority ranking associated with genes or genetic variants in the second set of genes and genetic variants is improved compared to a priority ranking associated with the first set of phenotype causing genes or genetic variants.

In some embodiments, the method further comprises using the programmed computer processor to integrate personal genomic data, gene function, and disease information with phenotype or disease description of an individual for improved accuracy to identify phenotype-causing variants or genes (Phevor). In some embodiments, the method further comprises using an algorithm that propagates information across and between ontologies. In some embodiments, the method further comprises accurately reprioritizing damaging genes or genetic variants identified in the first set of genes or genetic variants based on gene function, disease and phenotype knowledge. In some embodiments, the method further comprises incorporating a genomic profile of a single individual, wherein the genetic profile comprises single nucleotide polymorphisms, set of one or more genes, an exome or a genome, a genomic profile of one or more individuals analyzed together, or genomic profiles from individuals from a family. In some embodiments, the method improves diagnostic accuracy for individuals presenting with established disease phenotypes. In some embodiments, the method improves diagnostic accuracy for patients with novel or atypical disease presentations. In some embodiments, the method further comprises incorporating latent information in ontologies to discover new disease genes or disease causing-alleles.

In some embodiments, the first set of phenotype causing genes or genetic variants is identified by: using the computer processor to prioritize genetic variants by combining (1) variant prioritization information, (2) the knowledge resident in the one or more biomedical ontologies, and (3) a summing procedure; and automatically identifying and reporting the phenotype causing genes or genetic variants. In some embodiments, a phenotype description of sequenced individual(s) is included in the summing procedure. In some embodiments, the variant prioritization information is at least partially based on sequence characteristics selected from the group consisting of an amino acid substitution (AAS), a splice site, a promoters, a protein binding site, an enhancer, and a repressor. In some embodiments, the variant prioritization information is at least partially based on methods selected from the group consisting of VAAST, pVAAST, SIFT, ANNOVAR, burden-tests, and sequence conservation tools. In some embodiments, the one or more biomedical ontologies includes one or more of the Gene Ontology, Human Phenotype Ontology and Mammalian Phenotype Ontology. In some embodiments, the summing procedure comprises traversal of the ontologies, propagation of information across the ontologies and combination of one or more results of transversal and propagation, to produce a gene score which embodies a prior-likelihood that a given gene has an association with a user described phenotype or gene function.

In some embodiments, the variant prioritization information is performed using a variant protein impact score and/or frequency information. In some embodiments, the impact score is selected from the group consisting of SIFT, Polyphen, GERP, CADD, PhastCons and PhyloP.

In some embodiments, the phenotype description of the sequenced individual(s) is derived from a physical examination by a healthcare professional. In some embodiments, the phenotype description of the sequenced individual(s) is stored in an electronic medical health record. In some embodiments, the variants are prioritized in a genomic region comprising one or more genes or gene fragments, one or more chromosomes or chromosome fragments, one or more exons or exon fragments, one or more introns or intron fragments, one or more regulatory sequences or regulatory sequence fragments, or a combination thereof. In some embodiments, the biomedical ontologies are gene ontologies containing information with respect to gene function, process and location, disease ontologies containing information about human disease; phenotype ontologies containing knowledge concerning mutation phenotypes in non-human organisms, and information pertaining to paralogous and homologues genes and their mutant phenotypes in humans and other organisms.

In some embodiments, the sequenced individuals are of different species. In some embodiments, the phenotype is a disease. In some embodiments, family phenotype information on affected and non-affected individuals is included in the phenotype description.

In some embodiments, the method further comprises including set(s) of family genomic sequences. In some embodiments, the method further comprises incorporating a known inheritance mode.

In some embodiments, the method further comprises including sets of affected and non-affected genomic sequences. In some embodiments, the summing procedure is ontological propagation, and wherein seed nodes in some ontology are identified, each seed node is assigned a value greater than zero, and this information is propagated across the ontology. In some embodiments, the method further comprises proceeding from each seed node toward its children nodes, wherein when an edge to a neighboring node is traversed, a current value of a previous node is divided by a constant value. In some embodiments, the summing procedure is that upon completion of propagation , each node's value is renormalized to a value between zero and one by dividing by a sum of all nodes in the ontology. In some embodiments, (i) each gene annotated to an ontology receives a score corresponding to a maximum score of any node in the ontology to which that gene is annotated; and (ii) the method further comprises repeating (i) for each ontology, wherein genes annotated to a plurality of ontologies have a score from each ontology, and wherein scores from the plurality of ontologies are aggregated to produce a final sum score for each gene, and renormalized again to a value between one and zero.

In some embodiments, the sequenced individual(s) have genetic sequences that are from one or more cancer tissue and germline tissue. In some embodiments, the method further comprises (i) scoring both coding and non-coding variants; and (ii) evaluating a cumulative impact of both types of variants in the context of gene scores, wherein (1) the variants are prioritized in a genomic region comprising one or more genes or gene fragments, one or more chromosomes or chromosome fragments, one or more exons or exon fragments, one or more introns or intron fragments, one or more regulatory sequences or regulatory sequence fragments, or a combination thereof, and/or (2) the biomedical ontologies are gene ontologies containing information with respect to gene function, process and location, disease ontologies containing information about human disease; phenotype ontologies containing knowledge concerning mutation phenotypes in non-human organisms, and information pertaining to paralogous and homologues genes and their mutant phenotypes in humans and other organisms.

In some embodiments, the method further comprises incorporating both rare and common variants to identify variants responsible for common phenotypes. In some embodiments, the common phenotypes include a common disease.

In some embodiments, the method further comprises identifying rare variants causing rare phenotypes. In some embodiments, the rare phenotypes include a rare disease.

In some embodiments, the knowledge includes phenogenomic information. In some embodiments, the method has a statistical power at least 10 times greater than a statistical power of a method not using knowledge resident in one or more biomedical ontologies. In some embodiments, the method further comprises assessing a cumulative impact of variants in both coding and non-coding regions of a genome. In some embodiments, the method further comprises analyzing low-complexity and repetitive genome sequences. In some embodiments, the method further comprises analyzing pedigree data. In some embodiments, the method further comprises analyzing phased genome data. In some embodiments, family information on affected and non-affected individuals is included in a target and background database.

In some embodiments, the method is used in conjunction with a method for calculating a composite likelihood ratio (CLR) to evaluate whether a genomic feature contributes to a phenotype.

In some embodiments, the method further comprises calculating a disease association score (D_{g}) for each gene, wherein D_{g} = (1-V_{g}) × N_{g}, wherein N_{g} is a renormalized gene sum score derived from ontological propagation, and V_{g} is a percentile rank of a gene provided by the variant prioritization tool. In some embodiments, the method further comprises calculating a healthy association score (H_{g}) summarizing a weight of evidence that a gene is not involved with an illness of an individual, wherein, H_{g} = V_{g} × (1-N_{g}). In some embodiments, the method further comprises calculating a final score (Sg) as a log₁₀ ratio of disease association score (D_{g}) and the healthy association score (H_{g}), wherein S_{g} = log₁₀ D_{g}/H_{g.} In some embodiments, the method further comprises using a magnitude of S_{g} to re-rank or reprioritize each gene in the second set of phenotype causing genes or genetic variants.

In some embodiments, the user interface is a graphical user interface (GUI) of an electronic device of a user, which GUI has one or more graphical elements selected to display the second set of phenotype causing genes or genetic variants. In some embodiments, the user interface is a web-based user interface.

In some embodiments, the first and/or second set of phenotype causing genes or genetic variants are genetic markers. In some embodiments, the first set of phenotype causing genes or genetic variants is associated with a first set of ranking scores, the second set of phenotype causing genes or genetic variants is associated with a second set of ranking scores, wherein the second set of ranking scores is improved with respect to the first set of ranking scores.

In some embodiments, the method further comprises obtaining genetic information of a subject, and using the second set of phenotype causing genes or genetic variants to analyze the genetic information of the subject to identify a phenotype or disease condition in the subject. In some embodiments, the genetic information of the subject is obtained by sequencing, array hybridization or nucleic acid amplification using markers that are selected to identify the phenotype causing genes or genetic variants of the second set. In some embodiments, the method further comprises diagnosing a disease of the subject and/or recommending a therapeutic intervention for the subject. In some embodiments, the variant prioritization information of the first set of phenotype causing genes or genetic variants comprises use of family genomic sequences of affected or non-affected family members. In some embodiments, use of family genomic sequences comprises incorporating an inheritance mode based one or more of autosomal recessive, autosomal dominant, and x-lined.

In some embodiments, the method further comprises prioritizing and identifying disease causing genetic markers from a third set of phenotype causing genes or genetic variants based on the knowledge. In some embodiments, the method further comprises incorporating genomic profiles of one or more individuals, wherein the genomic profiles comprise measurements of one or more of the following: one or more single nucleotide polymorphisms, one or more genes, one or more exomes, and one or more genomes.

In some embodiments, a statistical power generated by the prioritizing analysis based on a combination of the one or more biomedical ontologies and genomic data is at least 10 times greater than a statistical power generated by the prioritizing analysis based on the one or more biomedical ontologies or the genomic data, but not both. In some embodiments, the method further comprises integrating the knowledge resident in one or more biomedical ontologies with an individual's phenotype or disease description to identify a third set of phenotype causing genes or genetic variants from the first and/or second sets of phenotype causing genes or genetic variants. In some embodiments, the third set of phenotype causing genes or genetic variants recognizes phenotype(s) with an improved accuracy measure with respect to the first and second sets of phenotype causing genes or genetic variants.

In some embodiments, the summing procedure is ontological propagation, and wherein one or more seed nodes are identified using one or more phenotype descriptions for a subject. In some embodiments, the one or more seed nodes are identified using a plurality of phenotype descriptions. In some embodiments, the method further comprises repeating (b)-(d) at least once using one or more different phenotype descriptions to yield an improved priority ranking.

In another aspect, the present disclosure provides a method for identifying phenotype-causing genetic variants, comprising (a) providing a computer processor coupled to computer memory that includes a plurality of phenotype causing genes or genetic variants, wherein the computer processor is programmed to identify and prioritize sets of phenotype causing genes or genetic variants among the plurality of phenotype causing genes or genetic variants; (b) using the computer processor to identify a first set of phenotype causing genes or genetic variants, which first set of phenotype causing genes or genetic variants is among the plurality of phenotype causing genes or genetic variants in the computer memory; (c) prioritizing the first set of phenotype causing genes or genetic variants based on knowledge resident in one or more biomedical ontologies; (d) automatically identifying a second set of phenotype causing genes or genetic variants, wherein a priority ranking associated with genes or genetic variants in the second set of genes and genetic variants is improved compared to a priority ranking associated with the first set of phenotype causing genes or genetic variants; and (e) using the second set of phenotype causing genes or genetic variants to analyze genetic information of a subject to identify a phenotype or disease condition in the subject.

In some embodiments, the method further comprises using the programmed computer processor to integrate personal genomic data, gene function, and disease information with phenotype or disease description of an individual for improved accuracy to identify phenotype-causing variants or genes (Phevor). In some embodiments, the first set of phenotype causing genes or genetic variants is identified by using the computer processor to prioritize genetic variants by combining (1) variant prioritization information, (2) the knowledge resident in the one or more biomedical ontologies, and (3) a summing procedure; and automatically identifying and reporting the phenotype causing genes or genetic variants. In some embodiments, the method further comprises obtaining the genetic information of the subject. In some embodiments, the genetic information of the subject is obtained by sequencing, array hybridization or nucleic acid amplification using markers that are selected to identify the phenotype causing genes or genetic variants of the second set. In some embodiments, the method further comprises diagnosing a disease of the subject and/or recommending a therapeutic intervention for the subject.

In another aspect, the present disclosure provides a computer-readable medium comprising machine executable code that, upon execution by one or more computer processors, implements any of the methods above or elsewhere herein.

In another aspect, the present disclosure provides a computer system comprising one or more computer processors and computer memory. The computer memory comprises machine executable code that, upon execution by the one or more computer processors, implements any of the methods above or elsewhere herein.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### INCORPORATION BY REFERENCE

All publications, patents, and patent applications mentioned in this specification are herein incorporated by reference to the same extent as if each individual publication, patent, or patent application was specifically and individually indicated to be incorporated by reference. To the extent publications and patents or patent applications incorporated by reference contradict the disclosure contained in the specification, the specification is intended to supersede and/or take precedence over any such contradictory material.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings (also "figure" and "FIG." herein), of which:
**FIG. 1** illustrates inputs to a phenotype driven variant ontological re-ranking tool (Phevor);
**FIG. 2** graphically illustrates combining ontologies;

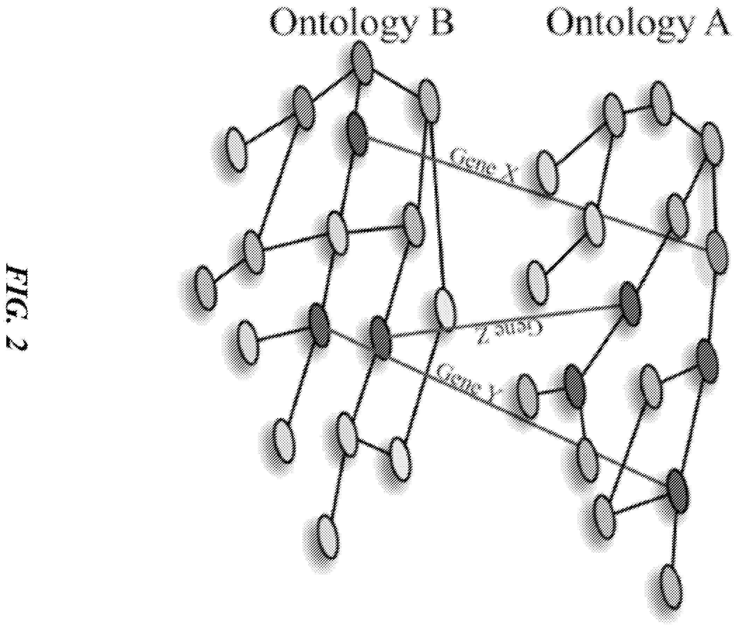

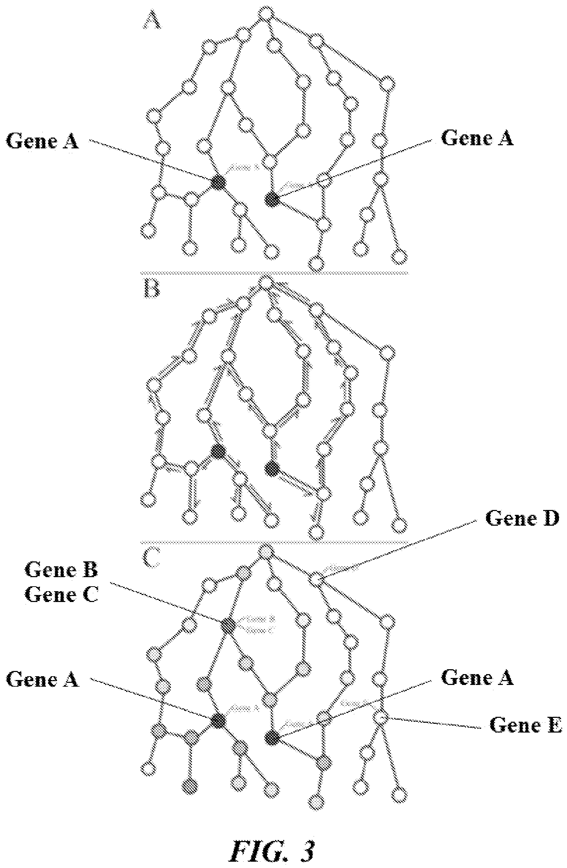

**FIG. 3** illustrates ontological propagation. Starting from a user-provided set of terms (nodes), supplemented by the cross-ontology linking procedure illustrated in **FIG. 2****,** Phevor next propagates this information across each ontology. **FIG. 3A** shows a hypothetical ontology, with two user-provided terms (nodes), marked by gene A. In this example, gene A has previously been annotated to both of these terms. This information is propagated across the ontology as illustrated in **FIG. 3B****.** First, these two 'seed nodes' are assigned a value of 1, and each time an edge is crossed to a neighboring node, the current value of the previous node is divided by 2. **FIG. 3C** illustrates the end result of the propagation process, with node colors corresponding to the magnitudes of their propagation scores, with darker nodes representing nodes with the greatest scores, white nodes with scores near zero. Note that nodes located at intersecting threads of propagation, far from the original seeds can attain high values, even exceeding those of the starting seed nodes. The phenomenon is illustrated by the darker nodes in **FIG. 3C****,** in which propagation has identified two additional gene-candidates, B and C not associated with the original seed nodes, but annotated to nodes with high propagation scores;
**FIG. 4** illustrates Variant Prioritization for Known Disease Genes. **FIG. 4A** shows performance comparisons of four different variant prioritization tools before processing with Phevor. **FIG. 4B** shows performance comparisons of four different variant prioritization tools after processing with Phevor;
**FIG. 5** illustrates variant prioritization for novel genes involved with known diseases;
**FIG. 6** illustrates a comparison of Phevor to exomiser (PHIVE);
**FIG. 7** schematically illustrates Phevor accuracy and atypical disease presentation;

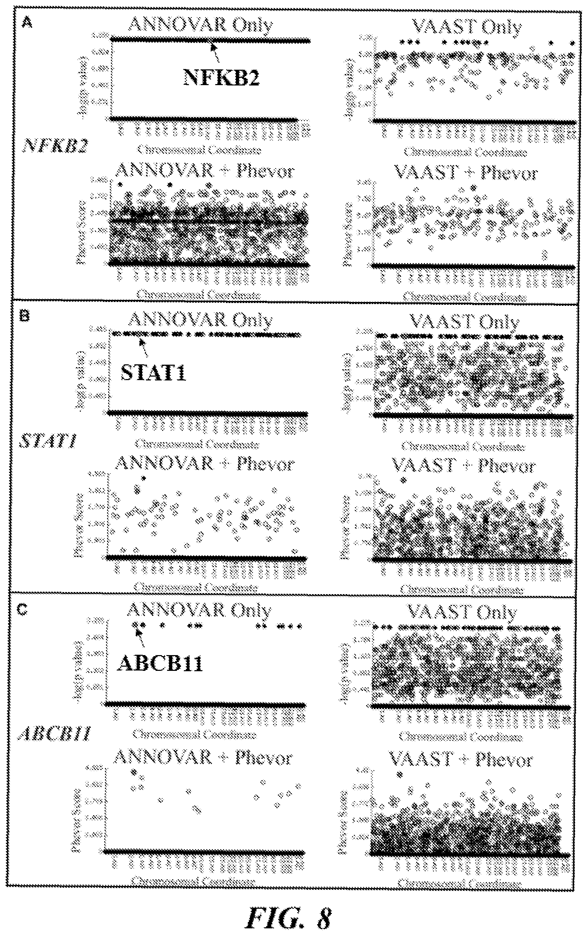

**FIG. 8** illustrates Phevor analyses of three clinical cases. Plotted on the x-axes of each Manhattan plot are the genomic coordinates of the candidate genes. The y-axes show the log₁₀ value of the Annovar score, Variant Annotation, Analysis and Search Tool (VAAST) p-value, or Phevor score depending upon panel. Black, filled circles denote top ranked gene(s), all having either the same Annovar score or VAAST p-value. Actual disease genes have been marked in select panels in the figures. For proposes of comparison to VAAST, the Annovar scores can be transformed to frequencies, dividing the number of gene-candidates identified by Annovar by the total number of annotated human genes. **FIG. 8A****. Phevor identifies NFKB2 as a new disease gene.** *Top.* Results of running Annovar (left) and VAAST (right) on the union of variants identified in an affected members of Family A, combined with those of affected individual from Family B. on the y-axis. Both Annovar and VAAST can identify a large number of equally likely candidate genes. NFKB2 (marked in top-left panel) is among them in both cases. *Bottom.* Phevor identifies a single best candidate, NFKB2, using the VAAST output, and NFKB2 is ranked second using the Annovar output, with two other genes tied for 1^{st} place. **FIG. 8B****. Phevor identifies a** ***de** novo* **variant in STAT1 as responsible for new phenotype in a known disease gene.** *Top.* Results of running Annovar (left) and VAAST (right) on the single affected are exome. Both Annovar and VAAST identify multiple candidate genes. STAT 1 (marked in top-left panel) is among them in both cases. *Bottom.* Phevor identifies a single best candidate, STAT1, using the VAAST output. STAT1 is the third best candidate using the Annovar output. FIG 8C. Phevor **identifies a new mutation in ABCB11, a known disease gene.** *Top.* Results of running Annovar (left) and VAAST (right) using the single affected child's exome. Both Annovar and VAAST identify a number of equally likely candidate genes. ABCB11 (marked in top-left panel) is among them. *Bottom.* Phevor identifies a single best candidate, ABCB11, using the Annovar and VAAST outputs;
**FIG. 9** illustrates variant prioritization for known disease genes (dominant). **FIG. 9A** shows performance comparisons of four different variant prioritization tools before Phevor. **FIG. 9B** shows performance comparisons of four different variant prioritization tools after Phevor;
**FIG. 10** shows a computer system that is programmed or otherwise configured to implement methods and systems of the present disclosure; and
**FIG. 11** shows a table with phenotype terms and descriptions used to create **FIGs. 4** **and** 9.

### DETAILED DESCRIPTION

The present disclosure may be understood more readily by reference to the following detailed description, the Examples included therein and to the Figures and their previous and following description.

Before the present methods are disclosed and described, it is to be understood that this disclosure is not limited to specific embodiments. It is also to be understood that the terminology used herein is for the purpose of describing particular embodiments only and is not intended to be limiting. The following description and examples illustrate some exemplary embodiments of the disclosure in detail. Those of skill in the art will recognize that there are numerous variations and modifications of this disclosure that are encompassed by its scope. Accordingly, the description of a certain exemplary embodiment should not be deemed to limit the scope of the present disclosure.

The term "subject," as used herein, generally refers to an animal, such as a mammalian species (e.g., human) or avian (e.g., bird) species, or other organism, such as a plant. A subject can be a vertebrate, a mammal, a mouse, a primate, a simian or a human. A subject can be a healthy individual, an individual that has or is suspected of having a disease or a pre-disposition to the disease, or an individual that is in need of therapy or suspected of needing therapy. A subject can be a patient.

An "individual" can be of any species of interest that comprises genetic information. The individual can be a eukaryote, a prokaryote, or a virus. The individual can be an animal or a plant. The individual can be a human or non-human animal.

The term "sequencing," as used herein, generally refers to methods and technologies for determining the sequence of nucleotide bases in one or more polynucleotides. The polynucleotides can be, for example, deoxyribonucleic acid (DNA) or ribonucleic acid (RNA), including variants or derivatives thereof (e.g., single stranded DNA). Sequencing can be performed by various systems currently available, such as, with limitation, a sequencing system by Illumina, Pacific Biosciences, Oxford Nanopore, or Life Technologies (Ion Torrent). Such devices may provide a plurality of raw genetic data corresponding to the genetic information of a subject (e.g., human), as generated by the device from a sample provided by the subject. In some situations, systems and methods provided herein may be used with proteomic information.

The term "genome," as used herein, generally refers to an entirety of an organism's hereditary information. A genome can be encoded either in deoxyribonucleic acid (DNA) or in ribonucleic acid (RNA). A genome can comprise regions that code for proteins as well as non-coding regions. A genome can include the sequence of all chromosomes together in an organism. For example, the human genome has a total of 46 chromosomes. The sequence of all of these together constitutes the human genome.

The term "variant," as used herein, generally refers to a genetic variant, such as a nucleic acid molecule comprising a polymorphism. A variant can be a structural variant or copy number variant, which can be genomic variants that are larger than single nucleotide variants or short indels. A variant can be an alteration or polymorphism in a nucleic acid sample or genome of a subject. Single nucleotide polymorphisms (SNPs) are a form of polymorphisms. Polymorphisms can include single nucleotide variations (SNVs), insertions, deletions, repeats, small insertions, small deletions, small repeats, structural variant junctions, variable length tandem repeats, and/or flanking sequences. Copy number variants (CNVs), transversions and other rearrangements are also forms of genetic variation. A genomic alternation may be a base change, insertion, deletion, repeat, copy number variation, or transversion.

A variant can be any change in an individual nucleotide sequence compared to a reference sequence. The reference sequence can be a single sequence, a cohort of reference sequences, or a consensus sequence derived from a cohort of reference sequences. An individual variant can be a coding variant or a non-coding variant. A variant wherein a single nucleotide within the individual sequence is changed in comparison to the reference sequence can be referred to as a single nucleotide polymorphism (SNP) or a single nucleotide variant (SNV), and these terms can be used interchangeably herein. SNPs that occur in the protein coding regions of genes that give rise to the expression of variant or defective proteins are potentially the cause of a genetic-based disease. Even SNPs that occur in non-coding regions can result in altered mRNA and/or protein expression. Examples are SNPs that defective splicing at exon/intron junctions. Exons are the regions in genes that contain three-nucleotide codons that are ultimately translated into the amino acids that form proteins. Introns are regions in genes that can be transcribed into pre-messenger RNA but do not code for amino acids. In the process by which genomic DNA is transcribed into messenger RNA, introns are often spliced out of pre-messenger RNA transcripts to yield messenger RNA. A SNP can be in a coding region or a non-coding region. A SNP in a coding region can be a silent mutation, otherwise known as a synonymous mutation, wherein an encoded amino acid is not changed due to the variant. An SNP in a coding region can be a missense mutation, wherein an encoded amino acid is changed due to the variant. An SNP in a coding region can also be a nonsense mutation, wherein the variant introduces a premature stop codon. A variant can include an insertion or deletion (indel) of one or more nucleotides. A variant can be a large-scale mutation in a chromosome structure; for example, a copy-number variant caused by an amplification or duplication of one or more genes or chromosome regions or a deletion of one or more genes or chromosomal regions; or a translocation causing the interchange of genetic parts from non-homologous chromosomes, an interstitial deletion, or an inversion.

Variants can be provided in a variant file, for example, a genome variant file (GVF) or a variant call format (VCF) file. The variant file can be in a memory location, such as a databse. According to the methods disclosed herein, tools can be provided to convert a variant file provided in one format to another more preferred format. A variant file can comprise frequency information on the included variants.

The term "read," as used herein, generally refers to a sequence of sufficient length (e.g., at least about 30 base pairs (bp)) that can be used to identify a larger sequence or region, e.g., that can be aligned to a location on a chromosome or genomic region or gene.

The term "coverage," as used herein, generally refers to the average number of reads representing a given nucleotide in a reconstructed sequence. Coverage can be calculated from the relationship N^{∗}L/G, wherein 'G' denotes the length of the original genome, 'N' denotes the number of reads, and 'L' denotes the average read length. For example, sequence coverage of 20 × means that each base in the sequence has been read 20 times.

The term "alignment," as used herein, generally refers to the arrangement of sequence reads to reconstruct a longer region of the genome. Reads can be used to reconstruct chromosomal regions, whole chromosomes, or the whole genome.

The term "indel," as used herein, generally refers to a class of mutations that include nucleotide insertions, deletions, or combinations thereof. In coding regions of the genome, an indel may cause a frameshift mutation, unless the length of the indel is a multiple of 3. Frameshift mutations can cause significant changes in the coding of amino acids that make up a polypeptide, often rendering the polypeptide nonfunctional. Frameshift mutations caused by indels can result in severe genetic disorders, e.g., Tay-Sachs Disease. An indel can be a frame-shift mutation, which can significantly alter a gene product. An indel can be a splice-site mutation.

The term "structural variant," as used herein, generally refers to a variation in structure of an organism's chromosome, such as greater than 1 kilobase (Kb) in length. Structural variants can comprise many kinds of variation in the genome, and can include, for example, deletions, duplications, copy-number variants, insertions, inversions and translocations, or chromosomal abnormalities. Typically a structure variation affects a sequence length about 1 Kb to 3 megabases (Mb), which is larger than SNPs and smaller than chromosome abnormality. In some cases, structural variants are associated with genetic diseases.

The term "calling," as used herein, generally refers to identification. For example, base calling is the identification of bases in a polynucleotide sequence. As another example, SNP calling is the identification of SNPs in a polynucleotide sequence. As another example, variant calling is the identification of variants in a genomic sequence.

"Nucleic acid" and "polynucleotide" can be used interchangeably herein, and refer to both RNA and DNA, including cDNA, genomic DNA, synthetic DNA, and DNA or RNA containing nucleic acid analogs. Polynucleotides can have any three-dimensional structure. A nucleic acid can be double-stranded or single-stranded (*e.g.,* a sense strand or an antisense strand). Non-limiting examples of polynucleotides include chromosomes, chromosome fragments, genes, intergenic regions, gene fragments, exons, introns, messenger RNA (mRNA), transfer RNA, ribosomal RNA, siRNA, micro-RNA, ribozymes, cDNA, recombinant polynucleotides, branched polynucleotides, nucleic acid probes and nucleic acid primers. A polynucleotide may contain unconventional or modified nucleotides.

"Nucleotides" are molecules that when joined together for the structural basis of polynucleotides, *e.g.,* ribonucleic acids (RNA) and deoxyribonucleic acids (DNA). A "nucleotide sequence" is the sequence of nucleotides in a given polynucleotide. A nucleotide sequence can also be the complete or partial sequence of a subject's genome and can therefore encompass the sequence of multiple, physically distinct polynucleotides (*e.g.,* chromosomes).

The "genome" of an individual member of a species can comprise that individual's complete set of chromosomes, including both coding and non-coding regions. Particular locations within the genome of a species are referred to as "loci", "sites" or "features". "Alleles" are varying forms of the genomic DNA located at a given site. In the case of a site where there are two distinct alleles in a species, referred to as "A" and "B", each individual member of the species can have one of four possible combinations: AA; AB; BA; and BB. The first allele of each pair is inherited from one parent, and the second from the other.

The "genotype" of a subject at a specific site in the subject's genome refers to the specific combination of alleles that the subject has inherited. A "genetic profile" for a subject includes information about the subject's genotype at a collection of sites in the subject's genome. As such, a genetic profile can be comprised of a set of data points, where each data point is the genotype of the subject at a particular site.

Genotype combinations with identical alleles (*e.g.,* AA and BB) at a given site are referred to as "homozygous"; genotype combinations with different alleles (*e.g.,* AB and BA) at that site are referred to as "heterozygous." It has to be noted that in determining the allele in a genome using standard techniques AB and BA cannot be differentiated, meaning it is impossible to determine from which parent a certain allele is inherited, given solely the genomic information of the subject tested. Moreover, variant AB parents can pass either variant A or variant B to their children. While such parents may not have a predisposition to develop a disease, their children may. For example, two variant AB parents can have children who are variant AA, variant AB, or variant BB. For example, one of the two homozygotic combinations in this set of three variant combinations may be associated with a disease. Having advance knowledge of this possibility can allow potential parents to make the best possible decisions about their children's health.

A subject's genotype can include haplotype information. A "haplotype" is a combination of alleles that are inherited or transmitted together. "Phased genotypes" or "phased datasets" provide sequence information along a given chromosome and can be used to provide haplotype information.

The term "phenotype," as used herein, generally refers to one or more characteristics of a subject. A phenotype of a subject can be the composite of the subject's observable characteristics, which may result from the expression of the subject's genes and, in some cases, the influence of environmental factors and the interactions between the two. A subject's phenotype can be driven by constituent proteins in the subject's "proteome," which is the collection of all proteins produced by the cells comprising the subject and coded for in the subject's genome. The proteome can also be defined as the collection of all proteins expressed in a given cell type within a subject. A disease or disease-state can be a phenotype and can therefore be associated with the collection of atoms, molecules, macromolecules, cells, tissues, organs, structures, fluids, metabolic, respiratory, pulmonary, neurological, reproductive or other physiological function, reflexes, behaviors and other physical characteristics observable in the subject through various approaches.

In many cases, a given phenotype can be associated with a specific genotype. For example, a subject with a certain pair of alleles for the gene that encodes for a particular lipoprotein associated with lipid transport may exhibit a phenotype characterized by a susceptibility to a hyperlipidemous disorder that leads to heart disease.

The term "background" or "background database," as used herein, generally refers to a collection of nucleotide sequences (*e.g.,* one or more genes or gene fragments, one or more chromosomes or chromosome fragments, one or more genomes or genome fragments, one or more transcriptome sequences, *etc.)* and their variants (variant files) used to derive reference variant frequencies in the background sequences. The background database can contain any number of nucleotide sequences and can vary based upon the number of available sequences. The background database can contain about 1-10000, 1-5000, 1-2500, 1-1000, 1-500, 1-100, 1-50, 1-10, 10-10000, 10-5000, 10-2500, 10-1000, 10-500, 10-100, 10-50, 50-10000, 50-5000, 50-2500, 50-1000, 50-500, 50-100, 100-10000, 100-5000, 100-2500, 100-1000, 100-500, 500-10000, 500-5000, 500-2500, 500-1000, 1000-10000, 1000-5000, 1000-2500, 2500-10000, 2500-5000, or 5000-10000 sequences, or any included sub-range; for example, about 1, 2, 3, 4, 5, 6, 7, 8, 9, 10, 15, 20, 25, 30, 35, 40, 45, 50, 60, 70, 80, 90, 100, 150, 200, 250, 300, 350, 400, 450, 500, 600, 700, 800, 900, 1000, 1250, 1500, 1750, 2000, 2500, 3000, 3500, 4000, 4500, 5000, 6000, 7000, 8000, 9000, 10000, or more sequences, or any intervening integer.

The term "target" or "case," as used herein, generally refers to a collection of nucleotide sequences (*e.g.,* one or more genes or gene fragments, one or more genomes or genome fragments, one or more transcriptome sequences, *etc.)* and their variants under study. The target can contain information from subjects that exhibit the phenotype under study. The target can be a personal genome sequence or collection of personal genome sequences. The personal genome sequence can be from a subject diagnosed with, suspected of having, or at increased risk for a disease. The target can be a tumor genome sequence. The target can be genetic sequences from plants or other species that have desirable characteristics.

The term "cohort," as used herein, generally refers to a collection of target or background sequences and their variants used in a given comparison. A cohort can include about 1-10000, 1-5000, 1-2500, 1-1000, 1-500, 1-100, 1-50, 1-10, 10-10000, 10-5000, 10-2500, 10-1000, 10-500, 10-100, 10-50, 50-10000, 50-5000, 50-2500, 50-1000, 50-500, 50-100, 100-10000, 100-5000, 100-2500, 100-1000, 100-500, 500-10000, 500-5000, 500-2500, 500-1000, 1000-10000, 1000-5000, 1000-2500, 2500-10000, 2500-5000, or 5000-10000 sequences, or any included sub-range; for example, about 1, 2, 3, 4, 5, 6, 7, 8, 9, 10, 15, 20, 25, 30, 35, 40, 45, 50, 60, 70, 80, 90, 100, 150, 200, 250, 300, 350, 400, 450, 500, 600, 700, 800, 900, 1000, 1250, 1500, 1750, 2000, 2500, 3000, 3500, 4000, 4500, 5000, 6000, 7000, 8000, 9000, 10000, or more sequences, or any intervening integer.

The term "feature," as used herein, generally refers to any span or a collection of spans within a nucleotide sequence (*e.g.,* a genome or transcriptome sequence). A feature can comprise a genome or genome fragment, one or more chromosomes or chromosome fragments, one or more genes or gene fragments, one or more transcripts or transcript fragments, one or more exons or exon fragments, one or more introns or intron fragments, one or more splice sites, one or more regulatory elements (*e.g.,* a promoter, an enhancer, a repressor, *etc.)* one or more plasmids or plasmid fragments, one or more artificial chromosomes or fragments, or a combination thereof. A feature can be automatically selected. A feature can be user-selectable.

The term "disease gene model," as used herein, generally refers to the mode of inheritance for a phenotype. A single gene disorder can be autosomal dominant, autosomal recessive, X-linked dominant, X-linked recessive, Y-linked, or mitochondrial. Diseases can also be multifactorial and/or polygenic or complex, involving more than one variant or damaged gene.

The term "pedigree," as used herein, generally refers to lineage or genealogical descent of a subject. Pedigree information can include polynucleotide sequence data from a known relative of a subject, such as a child, a sibling, a parent, an aunt or uncle, a grandparent, *etc.*

The term "amino acid" or "peptide," as used herein, generally refers to one of the twenty biologically occurring amino acids and to synthetic amino acids, including D/L optical isomers. Amino acids can be classified based upon the properties of their side chains as weakly acidic, weakly basic, hydrophilic, or hydrophobic. A "polypeptide" refers to a molecule formed by a sequence of two or more amino acids. Proteins are linear polypeptide chains composed of amino acid building blocks. The linear polypeptide sequence provides only a small part of the structural information that is important to the biochemist, however. The polypeptide chain folds to give secondary structural units (most commonly alpha helices and beta strands). Secondary structural units can then fold to give supersecondary structures (for example, beta sheets) and a tertiary structure. Most of the behaviors of a protein are determined by its secondary and tertiary structure, including those that are important for allowing the protein to function in a living system.

### Methods for identifying and prioritizing phenotype causing genes or genetic variants

An aspect of the present disclosure provides methods for the identification of phenotype-causing variants. The methods can comprise the comparison of polynucleotide sequences between a case, or target cohort, and a background, or control, cohort. Phenotype-causing variants can be scored within the context of one or more features. Variants can be coding or non-coding variants. The methods can employ a feature-based approach to prioritization of variants. The feature-based approach can be an aggregative approach whereby all the variants within a given feature are considered for their cumulative impact upon the feature (*e.g.,* a gene or gene product). Therefore, the method also allows for the identification of features such as genes or gene products. Prioritization can employ variant frequency information, sequence characteristics such as amino acid substitution effect information, phase information, pedigree information, disease inheritance models, or a combination thereof.

The present disclosure provides methods that integrate phenotype, gene function, and disease information with personal genomic data for improved power to identify disease-causing alleles. Such methods include a phenotype driven variant ontological re-ranking tool ("Phevor"). Phevor can combine knowledge resident in at least 1, 2, 3, 4, 5, 6, 7, 8, 9, 10, 20, 30, 40, or 50 biomedical ontologies with the outputs of variant prioritization tools. It can do so using an algorithm that propagates information across and between ontologies. This process enables Phevor to accurately reprioritize potentially damaging alleles identified by variant prioritization tools in light of the gene function, disease and phenotype knowledge. Phevor is especially useful for single exome and family trio-based diagnostic analyses, the most commonly occurring clinical scenarios, and ones for which existing personal-genomes diagnostic tools are most inaccurate and underpowered.

Also provided herein are a series of benchmark analyses illustrating Phevor's performance characteristics, including case studies in which Phevor is used to identify disease-causing alleles. Collectively, these results show that methods of the present disclosure, including Phevor, not only improve diagnostic accuracy for subjects (e.g., patients) presenting with established disease phenotypes, but also for subjects with novel and atypical disease presentations. Methods of the present disclosure, including Phevor, are not limited to known diseases or known disease-causing alleles. Such methods can also use latent information in ontologies to discover new disease genes and disease causing-alleles.

Personal genome sequencing is dramatically changing the landscape of clinical genetics, but it also presents a host of challenges. Every sequenced exome presents the clinical geneticist with thousands of variants, any one of which might be responsible for the patient's illness. One approach to analyzing these data is to employ a whole-genome/exome search tool such as Annovar [1] or VAAST [2, 3] to identify disease-causing variants in an *ab initio* fashion. This may be an effective approach for case-cohort analyses [4-8]; likewise, sequencing additional family members can also improve diagnostic accuracy. Unfortunately, single affected individuals and small nuclear families are the most frequently encountered diagnostic scenarios in the clinic. Today's variant prioritization tools may be underpowered in these situations, limiting the number of successful diagnoses [2, 9]. In response, physicians and clinical genetics laboratories often attempt to narrow the list to a subset of candidate genes and alleles in light of a patient's phenotype [10].

Patient phenotype data are generally employed in an *ad hoc* fashion with clinicians and geneticists choosing genes and alleles as candidates based upon their expert knowledge. No general standards, procedures or validated best practices are known. Moreover, genes not previously associated with the phenotype are not considered-often preventing novel discoveries. The potential impact of false positives and negatives on diagnostic accuracy is obviously considerable. Recognized herein is the need for computer implemented algorithms to prioritize genes and variants in light of patient phenotype data.

The present disclosure provides a phenotype driven variant ontological re-ranking tool (Phevor), which can be implemented by way of methods and systems provided herein. Phevor can combine the outputs of widely-used variant prioritization tools with knowledge resident in diverse biomedical ontologies, such as the Human Phenotype [11], the Mammalian Phenotype [12], the Disease [13] and the Gene [14] ontologies.

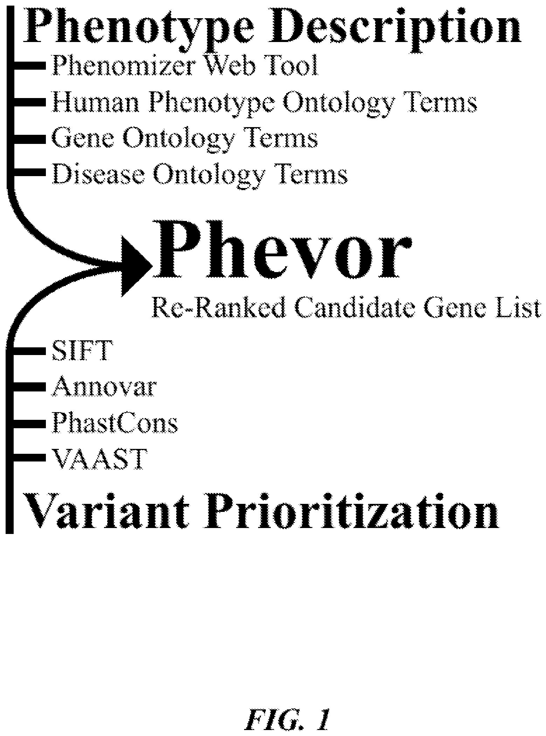

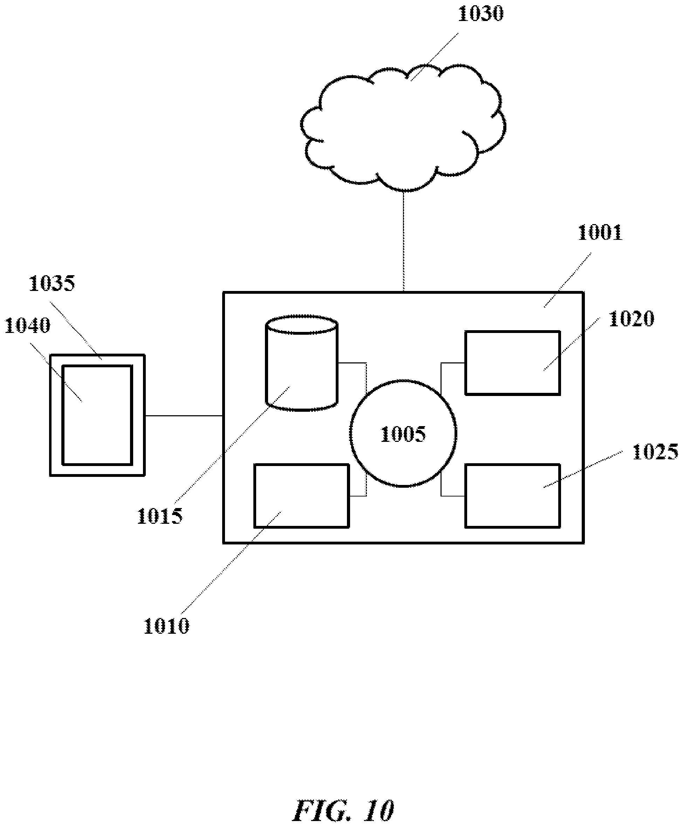

**FIG. 1** illustrates various inputs to Phevor. Phevor can be implemented using a computer system with computer memory and one or more programmed computer processors, as described elsewhere herein (see, e.g., **FIG. 10** and the corresponding text). Phevor can re-rank the outputs of variant prioritization tools in light of phenotype and gene function information. The inputs to Phevor are individual variant scores from tools such as Sorting Intolerant from Tolerant (SIFT) and PhastCons, candidate gene lists as returned by Annovar, or prioritized gene lists such as VAAST output files. These can be used together with a list of terms or their IDs describing the patient phenotype drawn from the Human Phenotype Ontology (HPO), the Disease Ontology (DO), the Mammalian Phenotype Ontology (MPO), or the Gene Ontology (GO). Mixtures of terms from more than one ontology are permitted, as are OMIM disease terms. Users may also employ the online tool Phenomizer to describe a patient phenotype and to assemble a list of candidate-genes.

Ontologies are graphical representations of the knowledge in a given domain, such as gene functions or human phenotypes. Ontologies organize this knowledge using directed acyclic graphs wherein concepts/terms are nodes in the graph and the logical relationships that obtain between them are modeled as edges, for example: *deaminase activity* (node) is_a (edge) *catalytic activity* (node) [14]. Ontology terms (nodes) can be used to 'annotate' biological data, rendering the data machine readable and traversable via the ontologies' relationships (edges). For example, annotating a gene with the term *deaminase activity* makes it possible to deduce that the same gene encodes a protein with *catalytic activity.* In recent years, many biomedical ontologies have been created for the management of biological data [15-17].

Phevor can propagate subject (e.g., patient) phenotype information across and between biomedical ontologies. This process can enable Phevor to accurately reprioritize candidates identified by variant prioritization tools in light of knowledge contained in the ontologies. Phevor can also discover emergent gene properties and latent phenotype information by combining ontologies, further improving its accuracy.

Phevor may not replace existing prioritization tools; rather, it can improve every tool's performance. As demonstrated herein, Phevor can substantially improve the accuracy of widely-used variant prioritization tools such as SIFT [18], conservation-based tools such as PhastCons [19], and genome-wide search tools such as Variant Annotation, Analysis and Search Tool (VAAST) [2, 3] and Annotate Variation (Annovar) [1]. Phevor also outperforms tools such as Phevor to exomiser (PHIVE) [20], which combine a fixed variant filtering approach with human and mouse phenotype data. PhastCons can function by fitting a two-state phylogenetic hidden Markov model (phylo-HMM) to data by maximum likelihood, subject to constraints designed to calibrate the model across species groups, and then predicting conserved elements based on this model.

Phevor can differ from tools such as Phenomizer [21] and sSAGA [10] in that it does not postulate a set of fixed associations between genes, phenotypes and diseases. Rather, Phevor dynamically integrates knowledge resident in multiple biomedical ontologies into the variant prioritization process. This enables Phevor not only to improve diagnostic accuracy for patients presenting with established disease phenotypes, but also for patients having novel and atypical disease presentations.

Phevor may not be limited to known disease-genes and known disease-causing alleles. Phevor can enable the integration of ontologies into the variant prioritization process, such as the Gene Ontology, which contain knowledge that has never before been explicitly linked to phenotype. As disclosed herein, Phevor can use information latent in such ontologies for discovery of new or otherwise unknown disease genes and disease causing-alleles.

Phevor is especially useful for single exome and family trio-based diagnostic analyses, the most commonly occurring clinical scenarios, and ones for which existing personal-genomes diagnostic tools are most inaccurate and underpowered.

The present disclosure describes an algorithm underlying Phevor. The present disclosure also present benchmark analyses illustrating Phevor's performance characteristics, and case studies in which Phevor is used to identify both known and novel (or otherwise unknown) disease-genes and disease-causing alleles.

Methods of the present disclosure can analyze personal genome sequence data. The input of the method can be a genome file. The genome file can comprise genome sequence files, partial genome sequence files, genome variant files (*e.g.,* VCF files, GVF files, *etc.),* partial genome variant files, genotyping array files, or any other DNA variant files. The genome variant files can contain the variants or difference of an individual genome or a set of genomes compared to a reference genome (*e.g.,* human reference assembly). These variant files can include variants such as single nucleotide variants (SNVs), single nucleotide polymorphisms (SNPs), small and larger insertion and deletions (indels), rearrangements, CNV (copy number variants), Structural Variants (SVs), *etc.* The variant file can include frequency information for each variant.

The methods disclosed herein can be used to identify, rank, and score variants by relevance either individually or in sets lying within a feature. A feature can be any span or a collection of spans on the genome sequence or transcriptome sequences such as a gene, transcript, exon, intron, UTRs, genetic locus or extended gene region including regulatory elements. A feature can also be a list of 2 or more genes, a genetic pathway or an ontology category.

The methods disclosed herein can be implemented as computer executable instructions or tools. In some embodiments, a computer readable medium comprises machine-executable code that upon execution by one or more computer processors implements any of the methods disclosed herein.

These analyses can be carried out on sets of genomes, making possible both pairwise (single against single genome, single against set of background genomes) and case-control style studies (set(s) of target genomes against set of background genomes) of personal genome sequences. Provided herein are several analyses of healthy and cancer genomes and show how variation hotspots can be identified both along the chromosome, and within gene ontologies, disease classes and metabolic pathways. Special emphasis can be placed upon the impact of data quality and ethnicity, and their consequences for further downstream analyses. Variant calling procedures, pseudogenes and gene families can all combine to complicate clinically-orientated analyses of personal genome sequences in ways that only become apparent when cohorts of genomes are analyzed.

In some embodiments, a method for identifying phenotype-causing genetic variants comprises providing a computer processor coupled to memory that includes a plurality of phenotype causing genes or genetic variants, wherein the computer processor is programmed to identify and prioritize sets of phenotype causing genes or genetic variants among the plurality of phenotype causing genes or genetic variants. Using the computer processor, a first set of phenotype causing genes or genetic variants among the plurality of phenotype causing genes or genetic variants is identified. Next, the first set of phenotype causing genes or genetic variants is prioritized based at least in part on knowledge resident in one or more biomedical ontologies. Next, a second set of phenotype causing genes or genetic variants is automatically identified and reported, such as on a user interface of an electronic device of a user. A priority ranking associated with genes or genetic variants in the second set of genes and genetic variants can be improved compared to a priority ranking associated with the first set of phenotype causing genes or genetic variants.

The method can further include incorporating latent information in ontologies to discover new disease genes or disease causing-alleles. This can permit the effective identification of disease genes that would otherwise not be identified.

The programmed computer processor can be used to integrate personal genomic data, gene function, and disease information with phenotype or disease description of an individual for improved accuracy to identify phenotype-causing variants or genes (Phevor). In some cases, an algorithm is used that propagates information across and between ontologies.

Damaging genes or genetic variants identified in the first set of genes or genetic variants can be re-prioritized based on gene function, disease and phenotype knowledge. A genomic profile of a single individual can be incorporated. The genetic profile can comprise single nucleotide polymorphisms, set of one or more genes, an exome or a genome, a genomic profile of one or more individuals analyzed together, or genomic profiles from individuals from a family.

The method can improve diagnostic accuracy for individuals presenting with established disease phenotypes. The method can improve diagnostic accuracy for patients with novel or atypical disease presentations.

The first set of phenotype causing genes or genetic variants can be identified by using the computer processor to prioritize genetic variants by combining (1) variant prioritization information, (2) the knowledge resident in the one or more biomedical ontologies, and (3) a summing (or other aggregation) procedure. Next, the phenotype causing genes or genetic variants are automatically identified and reported.

A phenotype description of sequenced individual(s) can be included in the summing procedure. The phenotype description can be an ICD9 or ICD10 number, in some examples. The phenotype description can have a level of detail from very specific to general description. The phenotype description can be a string of text, number(s) and symbol(s). The phenotype description can include one phenotype (e.g., "hypertension" or "short breath") or a plurality of phenotypes (e.g., "hypertension and short breath").

The sequenced individual(s) can have genetic sequences that are from one or more cancer tissue and germline tissue. The phenotype description of the sequenced individual(s) can be derived from a physical examination by a healthcare professional, such as a doctor. The phenotype description of the sequenced individual(s) can be stored in an electronic medical health record or database.

The variant prioritization information can be at least partially based on sequence characteristics selected from the group consisting of an amino acid substitution (AAS), a splice site, a promoters, a protein binding site, an enhancer, and a repressor. The variant prioritization information can be at least partially based on methods selected from the group consisting of VAAST, pVAAST, SIFT, ANNOVAR, burden-tests, and sequence conservation tools. VAAST can be as described in U.S. Patent Publication No. 2013/0332081 and Patent Cooperation Treaty (PCT) Publication No. WO/2012/034030, each of which is entirely incorporated herein by reference. The one or more biomedical ontologies can include one or more of the Gene Ontology, Human Phenotype Ontology and Mammalian Phenotype Ontology.

The summing procedure can include traversal of the ontologies, propagation of information across the ontologies and combination of one or more results of transversal and propagation, to produce a gene score which embodies a prior-likelihood that a given gene has an association with a user described phenotype or gene function. The variant prioritization information can be performed using a variant protein impact score and/or frequency information. In some examples, the impact score is selected from the group consisting of SIFT, Polyphen, GERP, CADD, PhastCons and PhyloP.

The variants can be prioritized in a genomic region comprising one or more genes or gene fragments, one or more chromosomes or chromosome fragments, one or more exons or exon fragments, one or more introns or intron fragments, one or more regulatory sequences or regulatory sequence fragments, or a combination thereof. The biomedical ontologies can be gene ontologies containing information with respect to gene function, process and location, disease ontologies containing information about human disease; phenotype ontologies containing knowledge concerning mutation phenotypes in non-human organisms, and information pertaining to paralogous and homologues genes and their mutant phenotypes in humans and other organisms.

The sequenced individuals can be of different species. As an alternative, the sequenced individuals can be of the same species (e.g., human).

The phenotype can be a disease or a collection of diseases. Family phenotype information on affected and non-affected individuals can be included in the phenotype description. In some cases, set(s) of family genomic sequences can be included. A known inheritance mode can be included. In some cases, the method further includes including sets of affected and non-affected genomic sequences.

The summing procedure can be an ontological propagation. Seed nodes in some ontology can be identified and each seed node can be assigned a value greater than zero. This information can then be propagated across the ontology. In some examples, this further includes proceeding from each seed node toward its children nodes. When an edge to a neighboring node is traversed, a current value of a previous node can be divided by a constant value. Upon completion of propagation, each node's value can be renormalized to a value between zero and one by dividing by a sum (or other aggregation) of all nodes in the ontology.

In some cases, one or more nodes are identified using one or more phenotype descriptions for a subject. At least some of the nodes can be seed nodes. For example, at least 1, 2, 3, 4, 5, 6, 7, 8, 9, or 10 nodes can be identified. The one or more nodes can be identified using a plurality of phenotype descriptions. In some cases, the method is repeated at least 1, 2, 3, 4, 5, 6, 7, 8, 9, 10, 20, 30, 40, 50, 100, 200, 300, 400, 500, or 1000 times using one or more different phenotype descriptions to yield an improved priority ranking.

In some cases, each gene annotated to an ontology receives a score corresponding to a maximum score of any node in the ontology to which that gene is annotated. This can be repeated for each ontology. Genes annotated to a plurality of ontologies have a score from each ontology, and wherein scores from the plurality of ontologies are aggregated to produce a final sum (or aggregation) score for each gene, and renormalized again to a value between one and zero.

In some cases, the method further includes (i) scoring both coding and non-coding variants, and (ii) evaluating a cumulative impact of both types of variants in the context of gene scores. In some cases, (1) the variants are prioritized in a genomic region comprising one or more genes or gene fragments, one or more chromosomes or chromosome fragments, one or more exons or exon fragments, one or more introns or intron fragments, one or more regulatory sequences or regulatory sequence fragments, or a combination thereof, and/or (2) the biomedical ontologies are gene ontologies containing information with respect to gene function, process and location, disease ontologies containing information about human disease; phenotype ontologies containing knowledge concerning mutation phenotypes in non-human organisms, and information pertaining to paralogous and homologues genes and their mutant phenotypes in humans and other organisms.

Both rare and common variants can be incorporated to identify variants responsible for common phenotypes. The common phenotypes can include a common disease.

This method can be used to identify rare variants causing rare phenotypes. The rare phenotypes can include a rare disease.

The knowledge resident in one or more biomedical ontologies can include phenogenomic information. Such information can be stored in a database. The database can be a local or remote database. The database can be publically accessible.

The method can have a statistical power at least 2, 3, 4, 5, 6, 7, 8, 9, 10, 50, or 100 times greater than a statistical power of a method not using the knowledge resident in one or more biomedical ontologies. The prioritizing, automatically identifying, or prioritizing and automatically identifying can have a statistical power at least 2, 3, 4, 5, 6, 7, 8, 9, 10, 50, or 100 times greater than a statistical power of prioritizing, automatically identifying, or prioritizing and automatically identifying by not using the knowledge resident in one or more biomedical ontologies. A statistical power generated by the prioritizing analysis based on a combination of the one or more biomedical ontologies and genomic data can be at least 2, 3, 4, 5, 6, 7, 8, 9, 10, 50, or 100 times greater than a statistical power generated by the prioritizing analysis based on the one or more biomedical ontologies or the genomic data, but not both.

The method can further include assessing a cumulative impact of variants in both coding and non-coding regions of a genome, and analyzing low-complexity and repetitive genome sequences and/or pedigree data. In some cases, phased genome data is analyzed.

Family information on affected and non-affected individuals can be included in a target and background database. In some cases, the method is used in conjunction with a method for calculating a composite likelihood ratio (CLR) to evaluate whether a genomic feature contributes to a phenotype.

The method can include calculating a disease association score (Dg) for each gene, wherein Dg = (1-Vg) x Ng, where Ng is a renormalized gene sum score derived from ontological propagation, and Vg is a percentile rank of a gene provided by the variant prioritization tool. Next, a healthy association score (Hg) can be calculated, which summarizes a weight of evidence that a gene is not involved with an illness of an individual, where Hg = Vg x (1-Ng). A final score (Sg) can then be calculated as a log₁₀ ratio of disease association score (Dg) and the healthy association score (Hg), wherein Sg = log₁₀ Dg/Hg. A magnitude of Sg can then be used to re-rank each gene in the second set of phenotype causing genes or genetic variants.

The user interface can be a graphical user interface (GUI) of an electronic device of a user. The GUI can h one or more graphical elements selected to display the second set of phenotype causing genes or genetic variants.

The first set of phenotype causing genes or genetic variants can be genetic markers. The second set of phenotype causing genes or genetic variants can be genetic markers. In some cases, one or more additional sets of phenotype causing genes or genetic variants can be used.

The first set of phenotype causing genes or genetic variants can be associated with a first set of ranking scores. The second set of phenotype causing genes or genetic variants can be associated with a second set of ranking scores. The second set of ranking scores can be improved with respect to the first set of ranking scores.

The method can include obtaining genetic information of a subject and using the second set of phenotype causing genes or genetic variants to analyze the genetic information of the subject to identify a phenotype or disease condition in the subject. In such a case, the second set of phenotype causing genes or genetic variants may not be reported on the user interface. The genetic information of the subject can be obtained by sequencing, array hybridization or nucleic acid amplification using markers that are selected to identify the phenotype causing genes or genetic variants of the second set. In some cases, the method further includes diagnosing a disease of the subject and/or recommending a therapeutic intervention for the subject. As an alternative, the method is performed without providing an immediate therapeutic intervention for the subject.

The variant prioritization information of the first set of phenotype causing genes or genetic variants can include use of family genomic sequences of affected or non-affected family members. The use of family genomic sequences can include incorporating an inheritance mode based one or more of autosomal recessive, autosomal dominant, and x-lined.

In some cases, disease causing genetic markers from a third set of phenotype causing genes or genetic variants based on the knowledge are identified. Such genetic markers can also be prioritized. The third set can be different than the first and/or second sets. In some cases, the third set is from a subject.

The method can further include incorporating genomic profiles of one or more individuals. The genomic profiles can comprise measurements of one or more of the following: one or more single nucleotide polymorphisms, one or more genes, one or more exomes, and one or more genomes.

The knowledge resident in one or more biomedical ontologies can be integrated with an individual's phenotype or disease description to identify a third set of phenotype causing genes or genetic variants from the first and/or second sets of phenotype causing genes or genetic variants. The third set of phenotype causing genes or genetic variants can recognize phenotype(s) with an improved accuracy measure (e.g., by at least about 5%, 10%, 20%, 30%, 40%, 50%, 80, 90%, or 100%) with respect to the first and second sets of phenotype causing genes or genetic variants. Such accuracy can be assessed by comparing application of the third set to an unknown data set to predict phenotype causing genes or genetic variants, and comparing such prediction to a known set of phenotype causing genes or genetic variants.

### Nucleotide Sequencing, Alignment, and Variant Identification

In an aspect, disclosed herein are methods of identifying and/or prioritizing phenotype causing variants utilizing nucleotide sequencing data. The methods can comprise comparing case and background sequencing information. Nucleotide sequencing information can be obtained using any known or future methodology or technology platform; for example, Sanger sequencing, dye-terminator sequencing, Massively Parallel Signature Sequencing (MPSS), Polony sequencing, 454 pyrosequencing, Illumina sequencing, SOLiD sequencing, ion semiconductor sequencing, DNA nanoball sequencing, sequencing by hybridization, or any combination thereof. Sequences from multiple different sequencing platforms can be used in the comparison. Non-limiting examples of types of sequence information that can be utilized in the methods disclosed herein are whole genome sequencing (WGS), exome sequencing, and exon-capture sequencing. The sequencing can be performed on paired-end sequencing libraries.

Sequencing data can be aligned to any known or future reference sequence. For example, if the sequencing data is from a human, the sequencing data can be aligned to a human genome sequence (*e.g.,* any current or future human sequence, *e.g.,* hg19 (GRCh37), hg18, hg17, hg16, hg15, hg13, hg12, hg11, hg8, hg7, hg6, hg5, hg4, *etc.).* (See hgdownload.cse.ucsc.edu/downloads.html). In one embodiment, the reference sequence is provided in a Fasta file. Fasta files can be used for providing a copy of the reference genome sequence. Each sequence (*e.g.,* chromosome or a contig) can begin with a header line, which can begin with the '>' character. The first contiguous set of non-whitespace characters after the '>' can be used as the ID of that sequence. In one embodiment, this ID must match the 'seqid' column described *supra* for the sequence feature and sequence variants. On the next and subsequent lines the sequence can be represented with the characters A, C, G, T, and N. In one embodiment, all other characters are disallowed. The sequence lines can be of any length. In one embodiment, all the lines must be the same length, except the final line of each sequence, which can terminate whenever necessary at the end of the sequence.

A General Feature Format version 3 (GFF3) file format can be used to annotate genomic features in the reference sequence. Although various versions of GTF and GFF formats have been in use for many years, GFF3 can be used to standardize the various gene annotation formats to allow better interoperability between genome projects. See www.sequenceontology.org/resources/gff3.html).

A GFF3 file can begin with one or more lines of pragma or meta-data information on lines that begin with '##'. In one embodiment, a required pragma is '## gff-version 3'. Header lines can be followed by one or more (usually many more) feature lines. In one embodiment, each feature line describes a single genomic feature. Each feature line can consist of nine tab-delimited columns. Each of the first eight columns can describe details about the feature and its location on the genome and the final line can be a set of tag value pairs that describe attributes of the feature.

A number of computer processor executable programs can be used to perform sequence alignments and the choice of which particular program to use can depend upon the type of sequencing data and/or the type of alignment required; for example, programs have been developed to perform a database search, conduct a pairwise alignment, perform a multiple sequence alignment, perform a genomics analysis, find a motif, perform benchmarking, and conduct a short sequence alignment. Examples of programs that can be used to perform a database search include BLAST, FASTA, HMMER, IDF, Infernal, Sequilab, SAM, and SSEARCH. Examples of programs that can be used to perform a pairwise alignment include ACANA, Bioconductor Biostrings::pairwiseAlignment, BioPerl dpAlign, BLASTZ, LASTZ, DNADot, DOTLET, FEAST, JAligner, LALIGN, mAlign, matcher, MCALIGN2, MUMmer, needle, Ngila, PatternHunter, ProbA (also propA), REPuter, Satsuma, SEQALN, SIM, GAP, NAP, LAP, SIM, SPA: Super pairwise alignment, Sequences Studio, SWIFT suit, stretcher, tranalign, UGENE, water, wordmatch, and YASS. Examples of programs that can be used to perform a multiple sequence alignment include ALE, AMAP, anon., BAli-Phy, CHAOS/DIALIGN, ClustalW, CodonCode Aligner, DIALIGN-TX and DIALIGN-T, DNA Alignment, FSA, Geneious, Kalign, MAFFT, MARNA, MAVID, MSA, MULTALIN, Multi-LAGAN, MUSCLE, Opal, Pecan, Phylo, PSAlign, RevTrans, Se-Al, StatAlign, Stemloc, T-Coffee, and UGENE. Examples of programs that can be used for genomics analysis include ACT (Artemis Comparison Tool), AVID, BLAT, GMAP, Mauve, MGA, Mulan, Multiz, PLAST-ncRNA, Sequerome, Sequilab, Shuffle-LAGAN, SIBsim4 / Sim4, and SLAM. Examples of programs that can be used for finding motifs include BLOCKS, eMOTIF, Gibbs motif sampler, HMMTOP, I-sites, MEME/MAST, MERCI, PHI-Blast, Phyloscan, and TEIRESIAS. Examples of programs that can be used for benchmarking include BAliBASE, HOMSTRAD, Oxbench, PFAM, PREFAB, SABmark, and SMART. Examples of software that can be used to perform a short sequence alignment include BFAST, BLASTN, BLAT, Bowtie, BWA, CASHX, CUDA-EC, drFAST, ELAND, GNUMAP, GEM, GMAP and GSNAP, Geneious Assembler, LAST, MAQ, mrFAST and mrsFAST, MOM, MOSAIK, MPscan, Novoalign, NextGENe, PALMapper, PerM, QPalma, RazerS, RMAP, rNA, RTG Investigator, Segemehl, SeqMap, Shrec, SHRiMP, SLIDER, SOAP, SOCS, SSAHA and SSAHA2, Stampy, SToRM, Taipan, UGENE, XpressAlign, and ZOOM. In one embodiment, sequence data is aligned to a reference sequence using Burroughs Wheeler alignment (BWA). Sequence alignment data can be stored in a SAM file. SAM (Sequence Alignment/Map) is a flexible generic format for storing nucleotide sequence alignment (see samtools.sourceforge.net/SAM1.pdf). Sequence alignment data can be stored in a BAM file, which is a compressed binary version of the SAM format (see genome.ucsc.edu/FAQ/FAQformat.html#format5.1). In one embodiment, sequence alignment data in SAM format is converted to BAM format.

Variants can be identified in sequencing data that has been aligned to a reference sequence using any known methodology. A variant can be a coding variant or a non-coding variant. A variant can be a single nucleotide polymorphism (SNP), also called a single nucleotide variant (SNV). Examples of SNPs in a coding region are silent mutations, otherwise known as a synonymous mutation; missense mutations, and nonsense mutations. A SNP in a non-coding region can alter a splice-site. A SNP in a non-coding region can alter a regulator sequence (*e.g.,* a promoter sequence, an enhancer seqeunce, an inhibiter sequence, *etc.).* A variant can include an insertion or deletion (indel) of one or more nucleotides. Examples of indels include frame-shift mutations and splice-site mutations. A variant can be a large-scale mutation in a chromosome structure; for example, a copy-number variant caused by an amplification or duplication of one or more genes or chromosome regions or a deletion of one or more genes or chromosomal regions; or a translocation causing the interchange of genetic parts from non-homologous chromosomes, an interstitial deletion, or an inversion.

Variants can be identified using SamTools, which provides various utilities for manipulating alignments in the SAM format, including sorting, merging, indexing and generating alignments in a per-position format (see samtools.sourceforge.net). In one embodiment, variants are called using the mpileup command in SamTools. Variants can be identified using the Genome Analysis Toolkit (GATK) (see www.broadinstitute.org/gsa/wiki/index.php/The_Genome_Analysis_Toolkit). In one embodiment, regions surrounding potential indels can be realigned using the GATK IndelRealigner tool. In one embodiment, variants are called using the GATK UnifiedGenotypeCaller and IndelCaller. Variants can be identified using the Genomic Next-generation Universal MAPer (GNUMAP) program (see dna.cs.byu.edu/gnumap/). In one embodiment, GNUMAP is used to align and/or identify variants in next generation sequencing data.

### Variant Files

In one aspect, disclosed herein are methods of identifying and/or prioritizing phenotype causing variants, wherein the variants are provided in one or more variant files. The methods can comprise comparing a target cohort of variants to a background cohort of variants. The variants can be provided in one or more variant files. Non-limiting examples of variant file formats are genome variant file (GVF) format and variant call format (VCF). The GVF file format is introduced by the Sequence Ontology group for use in describing sequence variants. It is based on the GFF3 format and is fully compatible with GFF3 and tools built for parsing, analyzing and viewing GFF3. (See www.sequenceontology.org/gvf.html). GVF shares the same nine-column format for feature lines, but specifies additional pragmas for use at the top of the file and additional tag/value pairs to describe feature attributes in column nine that are specific to variant features (e.g., variant effects). According to the methods disclosed herein, tools can be provided to convert a variant file provided in one format to another format. In one embodiment, variant files in VCF format are converted to GVF format using a tool called vaast_converter. In one embodiment, variant effect information is added to a GVF format file using a variant annotation tool (VAT). A variant file can comprise frequency information on the included variants.

### Target and Background Cohorts

In one aspect, disclosed herein are methods of identifying and/or prioritizing phenotype causing variants by comparing a target cohort of variants to a background cohort of variants. A cohort is defined as a grouping of one or more individuals. A cohort can contain any number of individuals; for example, about 1-10000, 1-5000, 1-2500, 1-1000, 1-500, 1-100, 1-50, 1-10, 10-10000, 10-5000, 10-2500, 10-1000, 10-500, 10-100, 10-50, 50-10000, 50-5000, 50-2500, 50-1000, 50-500, 50-100, 100-10000, 100-5000, 100-2500, 100-1000, 100-500, 500-10000, 500-5000, 500-2500, 500-1000, 1000-10000, 1000-5000, 1000-2500, 2500-10000, 2500-5000, or 5000-10000 individuals, or any included sub-range. A cohort can contain about 1, 2, 3, 4, 5, 6, 7, 8, 9, 10, 15, 20, 25, 30, 35, 40, 45, 50, 60, 70, 80, 90, 100, 150, 200, 250, 300, 350, 400, 450, 500, 600, 700, 800, 900, 1000, 1250, 1500, 1750, 2000, 2500, 3000, 3500, 4000, 4500, 5000, 6000, 7000, 8000, 9000, 10000, or more individuals, or any intervening integer. The target cohort can contain information from the individual(s) under study (*e.g.,* individuals that exhibit the phenotype of interest). The background cohort contains information from the individual(s) serving as healthy controls.

### Selection of variants within a cohort

The target and/or background cohorts can contain a variant file corresponding to each of the individuals within the cohort. The variant file(s) can be derived from individual sequencing data aligned to a reference sequence. The variant files can be in any format; non limiting examples including the VCF and GVF formats. In one embodiment, a set of variants from the individual variant files in a target or background cohort are combined into a single, condensed variant file. A number of options for producing a set of variants in a condensed variant file can be used. The condensed variant file can contain the union of all of the individual variant files in a cohort, wherein the set of variant in the condensed variant file contains all the variants found in the individual files. The condensed variant file can contain the intersection of all individual variant files in a cohort, wherein set of variants in the condensed variant file contains only those variants that are common to all of the individual variant files. The condensed variant file can contain the compliment of the individual variant files, wherein set of variants in the condensed variant file contains the variants that are unique to a specified individual variant file within the cohort of individual variant files. The condensed variant file can contain the difference of the individual variant files, wherein the set of variants in the condensed variant file contains all of the variants that unique to any of the individual variant files. The condensed variant file can contain the variants that are shared between a specified number of individual files. For example, if the specified number is 2, then the set of variants in the condensed variant file can contain only those variants that are found in at least two individual variant files. The specified number of variant files can be between 2 and N, wherein N is the number of individual variant files in a cohort. In one embodiment, a subset of the individual variant files can be specified and combined into a condensed variant file using any of these described methods. More than one method of combining individual variant files can be used to produce a combined variant file. For example, a combined variant file can be produced that contains the set of variants found in one group of the cohort but not another group of the cohort. In one embodiment, a software tool is provided to combine variant files into a condensed variant file. In one embodiment, the software tool is the Variant Selection Tool (VST).

### Computer systems

The present disclosure provides computer control systems that are programmed to implement methods of the disclosure. **FIG. 10** shows a computer system 1001 that is programmed or otherwise configured to implements methods of the present disclosure. The computer system 1001 can regulate various aspects of methods of the present disclosure, such as, for example, methods that integrate phenotype, gene function, and disease information with personal genomic data for improved power to identify disease-causing alleles (Phevor). The computer system 1001 can be an electronic device of a user or a computer system that is remotely located with respect to the electronic device. The electronic device can be a mobile electronic device. As an alternative, the computer system 1001 can be a computer server.

The computer system 1001 includes a central processing unit (CPU, also "processor" and "computer processor" herein) 1005, which can be a single core or multi core processor, or a plurality of processors for parallel processing. The computer system 1001 also includes memory or memory location 1010 (e.g., random-access memory, read-only memory, flash memory), electronic storage unit 1015 (e.g., hard disk), communication interface 1020 (e.g., network adapter) for communicating with one or more other systems, and peripheral devices 1025, such as cache, other memory, data storage and/or electronic display adapters. The memory 1010, storage unit 1015, interface 1020 and peripheral devices 1025 are in communication with the CPU 1005 through a communication bus (solid lines), such as a motherboard. The storage unit 1015 can be a data storage unit (or data repository) for storing data. The computer system 1001 can be operatively coupled to a computer network ("network") 1030 with the aid of the communication interface 1020. The network 1030 can be the Internet, an internet and/or extranet, or an intranet and/or extranet that is in communication with the Internet. The network 1030 in some cases is a telecommunication and/or data network. The network 1030 can include one or more computer servers, which can enable distributed computing, such as cloud computing. The network 1030, in some cases with the aid of the computer system 1001, can implement a peer-to-peer network, which may enable devices coupled to the computer system 1001 to behave as a client or a server.

The CPU 1005 can execute a sequence of machine-readable instructions, which can be embodied in a program or software. The instructions may be stored in a memory location, such as the memory 1010. The instructions can be directed to the CPU 1005, which can subsequently program or otherwise configure the CPU 1005 to implement methods of the present disclosure. Examples of operations performed by the CPU 1005 can include fetch, decode, execute, and writeback.

The CPU 1005 can be part of a circuit, such as an integrated circuit. One or more other components of the system 1001 can be included in the circuit. In some cases, the circuit is an application specific integrated circuit (ASIC).

The storage unit 1015 can store files, such as drivers, libraries and saved programs. The storage unit 1015 can store user data, e.g., user preferences and user programs. The computer system 1001 in some cases can include one or more additional data storage units that are external to the computer system 1001, such as located on a remote server that is in communication with the computer system 1001 through an intranet or the Internet.

The computer system 1001 can communicate with one or more remote computer systems through the network 1030. For instance, the computer system 1001 can communicate with a remote computer system of a user (e.g., patient, healthcare provider, or service provider). Examples of remote computer systems include personal computers (e.g., portable PC), slate or tablet PC's (e.g., Apple^{®} iPad, Samsung^{®} Galaxy Tab), telephones, Smart phones (e.g., Apple^{®} iPhone, Android-enabled device, Blackberry^{®}), or personal digital assistants. The user can access the computer system 1001 via the network 1030.

Methods as described herein can be implemented by way of machine (e.g., computer processor) executable code stored on an electronic storage location of the computer system 1001, such as, for example, on the memory 1010 or electronic storage unit 1015. The machine executable or machine readable code can be provided in the form of software. During use, the code can be executed by the processor 1005. In some cases, the code can be retrieved from the storage unit 1015 and stored on the memory 1010 for ready access by the processor 1005. In some situations, the electronic storage unit 1015 can be precluded, and machine-executable instructions are stored on memory 1010.

The code can be pre-compiled and configured for use with a machine having a processer adapted to execute the code, or can be compiled during runtime. The code can be supplied in a programming language that can be selected to enable the code to execute in a pre-compiled or as-compiled fashion.

Aspects of the systems and methods provided herein, such as the computer system 1001, can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such as memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system 1001 can include or be in communication with an electronic display 1035 that comprises a user interface (UI) 1040 for providing, for example, genetic information, such as an identification of disease-causing alleles in single individuals or groups of individuals. Examples of UI's include, without limitation, a graphical user interface (GUI) and web-based user interface (or web interface).

Methods and systems of the present disclosure can be implemented by way of one or more algorithms. An algorithm can be implemented by way of software upon execution by the central processing unit 1005. The algorithm can, for example, implement methods that integrate phenotype, gene function, and disease information with personal genomic data for improved power to identify disease-causing alleles (Phevor).

### EXAMPLES

Examples illustrating various methods and systems of the present disclosure will now be discussed. It will be appreciated that such examples are illustrative of various methods and systems of the present disclosure and are not intended to be limiting.

**Phenotype and candidate-gene information.** Phevor can improve diagnostic accuracy using patient phenotype and candidate-gene information derived from multiple sources. In the simplest scenario, users provide a tab-delimited list of terms describing the patient(s) phenotype(s) drawn from the Human Phenotype Ontology (HPO) [11]. Alternatively, the list can include terms from the Disease Ontology (DO) [13], the Mammalian Phenotype Ontology (MPO) [12], the Gene Ontology [14] or OMIM disease terms [22]. Lists containing terms from more than one ontology are also permitted. Users may also employ the online tool Phenomizer [21] to describe a patient phenotype and to assemble a list of candidate-genes. The Phenomizer report can be downloaded to the user's computer and passed directly to Phevor.

**Assembling a gene list.** Biomedical ontology annotations are now readily available for many human and model organism genes. An example is the Gene Ontology (GO). Currently over 18,000 human genes have been annotated with GO terms [14]. In addition, at last count over 2500 known human disease genes have been annotated with HPO terms [11]. Phevor can employ these annotations to associate ontology concepts (nodes) to genes, and vice versa. Consider the following example of a patient phenotype description consisting of two HPO terms: *Hypothyroidism* (HP:0000812) and *Abnormality of the intestine* (HP:0002242). If genes have previously been annotated to these two nodes in the ontology, Phevor saves those genes in an internal list (e.g., in computer memory). In cases where no genes are annotated to a user-provided ontology term, Phevor traverses that ontology beginning at the provided term and proceeds toward the ontology's root(s) until it encounters a node with annotated genes, adding those genes to the list. At the end of this process, the resulting gene list is then used to seed nodes in the other ontologies, the Gene Ontology (GO), the Mammalian Phenotype Ontology (MPO) and the Disease Ontology (DO), for example.

Phevor can relate different ontologies via their common gene annotations. **FIG. 2** illustrates combining gene ontologies. Phevor can relate different ontologies via their common gene annotations. **FIG. 2** shows two generic ontologies, Ontology A and Ontology B. Circles denote terms, or 'nodes', with edges denoting relationships between terms. For purposes of illustration, assume that each edge is directed, with the root of both ontologies lying at the top left-hand end of the graph. The blue lines connecting the two ontologies represent three different genes X, Y and Z that are annotated to both ontologies. Phevor uses genes that have been annotated to two or more ontologies to relate terms in ontology A to those in B and vice versa. This cross-ontology linking procedure allows Phevor to combine knowledge from different ontological domains, e.g., phenotype information from HPO and gene function, process and location information from GO.

For example, deleterious alleles in the ABCB11 gene are known to cause Intrahepatic Cholestasis, a fact captured by HPO's annotation of the ABCB11 gene to the node HP:0001406 *(Intrahepatic Cholestasis).* In GO, ABCB11 is annotated to *canalicular bile acid transport* (GO:0015722) and *bile acid biosynthetic process* (GO:0006699). Phevor uses the common gene (in this case ABCB11) to relate the HPO node HP:0001406 to GO nodes GO:0015722 and GO:0006699. This process can allow Phevor to extend its search to include additional genes with functions similar to ABCB11, as described elsewhere herein. This can advantageously permit the discovery of new relationships, new disease genes and disease causing-alleles that would otherwise not be possible.

**Ontology Propagation.** Once a set of starting nodes for each ontology has been identified, i.e., those provided by the user in their phenotype list (e.g., HP:0001406), or derived from it by the cross-ontology linking procedure described in the preceding paragraph (e.g., GO:0015722 and GO:0006699), Phevor can subsequently propagate this information across each ontology using an ontological propagation process. With reference to **FIG. 3A****,** two seed nodes in some ontology have been identified; in both cases, gene A has been previously annotated to both nodes. Each seed node is assigned a value of 1 and this information is then propagated across the ontology as follows. Proceeding from each seed node toward its children, each time an edge is crossed to a neighboring node, the current value of the previous node is divided by a constant value, such as 2. For example, if the starting seed node has two children, its value is divided in half for each child, so in this case, both children receive a value of 1/2. This process is continued until a terminal leaf is encountered. The original seed scores are also propagated upwards to the root node(s) of the ontology using the same procedure **(****FIG. 3B****).** In practice there can be many seed nodes. In such cases intersecting threads of propagation are first combined by adding them, and the process of propagation proceeds as previously described. One interesting consequence of this process is that nodes far from the original seeds can attain high values, greater even than any of the starting seed nodes. The phenomenon is illustrated by the darker nodes (marked by Gene A, Gene B and Gene C) in **FIG. 3C****,** in which propagation has identified two additional gene-candidates, B and C not associated with the original seed nodes.

**From node to gene.** Upon completion of propagation **(****FIG. 3C****),** each node's value is renormalized to a value between zero and one by dividing it by the sum of all nodes in the ontology. Phevor next assigns each gene annotated to the ontology a score corresponding to the maximum score of any node in the ontology to which it is annotated. This process is repeated for each ontology. Genes annotated to more than one ontology will have a score from each ontology. These scores are added (or aggregated) to produce a final sum score for each gene, and renormalized again to a value between one and zero.

Consider a set of known disease genes drawn from HPO and assigned gene scores by the process described in the preceding paragraphs. Consider also a similar list of human genes derived from propagation across GO. Summing each gene's HPO and GO scores and renormalizing again by the total sum of sums will combine these lists.

**Rational candidate-gene list expansion.** The ontological propagation and combination procedures described above enable Phevor to extend the original HPO-derived gene list into an expanded candidate-gene list that can also include genes not annotated to the HPO. Recall that during propagation across an ontology, intersecting threads can result in nodes having scores that equal or even exceed those of any original seed nodes. Thus a gene not yet associated with a particular human disease can become an excellent candidate, because it is annotated to an HPO node located at an intersection of phenotypes associated with other diseases, or has GO functions, locations and/or processes similar to those of known disease-genes annotated to HPO. Phevor also employs the Mammalian Ontology, allowing it to leverage model organism phenotype information, and the Disease Ontology, which provides it with additional information pertaining to human genetic disease. Thus Phevor's approach enables an automatic and rational expansion of a candidate disease-gene list derived from a starting list of phenotype terms, one that leverages knowledge contained in diverse biomedical ontologies. Gene sum scores can be combined with variant prioritization tools to improve the accuracy of sequence-based patient diagnosis, as described elsewhere herein.

**Combining ontologies and variant data.** Upon completion of all ontology propagation, combination and gene scoring steps described in the preceding paragraphs, genes are ranked using their gene sum scores; then their percentile ranks are combined with variant and gene prioritization scores as follows. Phevor first calculates a disease association score for each gene using the relationship D_{g} = (1 - V_{g}) × N_{g} (Equation 1), where N_{g} is the renormalized gene sum score derived from the ontological combination propagation procedures described in **FIGs. 2** **and** **3****,** and V_{g} is the percentile rank of the gene provided by the external variant prioritization tool, e.g., Annovar, SIFT and PhastCons (except for VAAST, in which case its reported p-values can be used directly). Phevor then calculates a second score summarizing the weight of evidence that the gene is not involved with the patient's illness, H_{g}, i.e., neither the variants nor the gene are involved in the patient's disease, using the relationship H_{g} = V_{g} × (1 - N_{g}) (Equation 2). The Phevor score (S_{g}) is the log₁₀ ratio of disease association score (D_{g}), and the healthy association score (H_{g}), given by the relationship S_{g} = log₁₀ D_{g}/H_{g} (Equation 3). These scores are distributed normally (data not shown). The performance benchmarks presented in the Results and Discussion section provide an objective basis for evaluating the utility of S_{g}.

**Sequencing procedures.** For exome DNA sequencing, an Agilent SureSelect(XT) Human All Exon v5 plus UTRs targeted enrichment system is used. The STAT proband's (see results and Discussion for details), whole genome is sequenced. An Illumina HiSeq instrument programmed to perform 101 cycle paired sequencing is used for all cases.

**Sanger sequence validation.** Putative disease-causing mutations identified by exome sequencing are validated by Sanger sequencing. See, e.g., Sanger F, Coulson AR (May 1975), "A rapid method for determining sequences in DNA by primed synthesis with DNA polymerase," J. Mol. Biol. 94 (3): 441-8, and Sanger F, Nicklen S, Coulson AR (December 1977), "DNA sequencing with chain-terminating inhibitors," Proc. Natl. Acad. Sci. U.S.A. 74 (12): 5463-7, which are entirely incorporated herein by reference. DNA from probands and parents is also used to validate inheritance patterns or confirm *de novo* mutations. Polymerase chain reaction primers are designed and optimized and subsequently amplified. Sequencing is performed using capillary sequencing.

**Variant calling procedures.** Following the best practices described by the Broad Institute [23], sequence reads are aligned using BWA, PCR duplicates are removed and indel realignment is performed using the GATK. Variants are joint called using the GATK UnifiedGenotyper in conjunction with 30 CEU Genome BAM files from the 1000 Genomes Project [24]. For the benchmarking experiments only SNV variants can be used, because not every variant prioritization tool can score indels and splice-site variants. The case study analyses searched SNVs, splice-site and Indel variants.

**Benchmarking procedures.** Known, disease-causing alleles are inserted in otherwise healthy (background) exomes. These exomes are sequenced to 50x coverage on an Illumina HiSeq (see sequencing procedures above) and jointly called with 30 CEU genomes drawn from the 1000 genomes project [24]. Known disease-genes are randomly selected (without replacement) from a gene mutation database (e.g., the Human Gene Mutation Database). For each disease-gene, damaging SNV alleles are randomly selected (without replacement) from all recorded damaging alleles ("DM" alleles) at that locus. The damaging allele is added to the target exome(s) VCF [25] file(s) and the quality metrics of the closest mapped variant are attached to it. Damaging alleles are inserted into the appropriate number of healthy exomes depending upon inheritance model (e.g., two copies of the same allele for recessive, one for dominant). This process is repeated 100 times for 100 different, randomly selected known disease genes, with this entire process then repeated 99 more times in order to determine margins of error. All prioritization tools (SIFT, PhastCons, Annovar and VAAST) are run using their default settings, except that dominant or recessive inheritance is specified for the VAAST and Annovar runs, as these two tools allow users to do so. For the VAAST and Annovar runs, the max allele MAF is set to 1%. Annovar may also be run with different MAF allele cutoffs, but overall performance may be best using this value. Annovar is run with the clinical variant flag enabled, so as not to exclude known disease-causing variants present in dbSNP 135 from consideration. PHIVE [20] can be run using the Exomiser web-server, which is accessible over the Internet. For these runs, the MAF is set to 1% and the remover ad dbSNP and pathogenic variant flags options are set to 'no'.

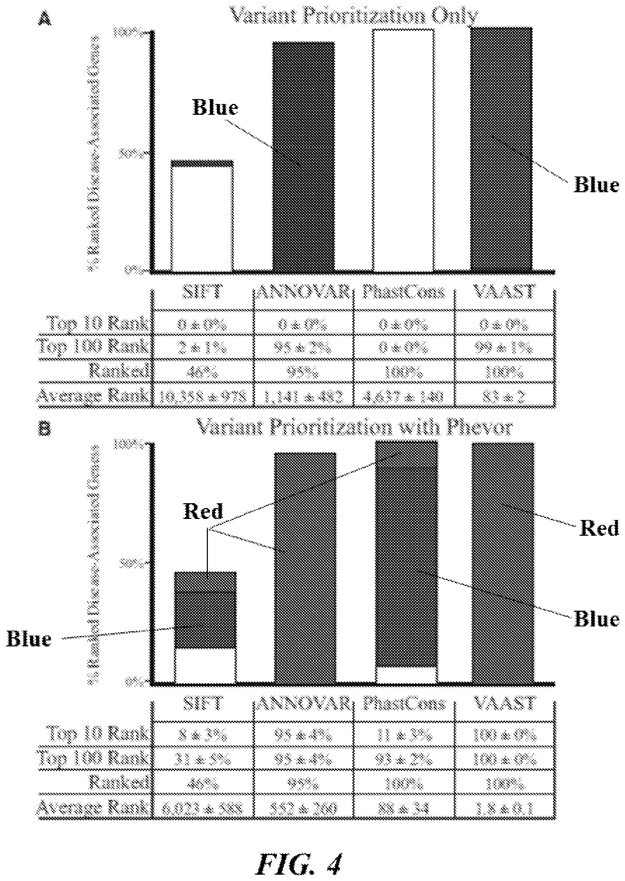

**FIG. 4** illustrates variant prioritization for known disease genes. This figure shows performance comparisons of four different variant prioritization tools before (top panel, **FIG 4A**)**,** and after post-processing them with Phevor (bottom panel, **FIG 4B**)**.** Two copies of a known disease-causing allele are randomly selected from HGMD and spiked into a single target exome at the reported genomic location; hence these results model simple, recessive diseases. This process is repeated 100 times for 100 different, randomly selected known disease genes in order to determine margins of error. Bar charts show the percentage of time the disease gene is ranked among the top ten candidates genome-wide (red), or among the top 100 candidates (blue), with white (color not labeled) denoting a rank greater than 100 in the candidate list. For the Phevor analyses shown in the bottom panel, each tool's output files are fed to Phevor along with phenotype report containing the HPO terms annotated to each disease gene. The table below the bar charts summarizes this information in more detail. Bars do not reach 100% due to false negatives, i.e., the tool is unable to prioritize the disease-causing allele. Damaging alleles predicted to be benign are placed at the midpoint of the list 22,107 annotated human genes.

The top panel of **FIG. 4** summarizes the ability of four different variant prioritization tools, SIFT, Annovar, PhastCons and VAAST to identify recessive disease alleles within a known disease-gene using a single affected individual's exome. These four tools are selected to represent prominent classes of variant prioritization tools. SIFT [18] is an amino acid conservation and functional prediction tool, PhastCons [19] is a sequence-conservation identification tool, Annovar [1] filters on variant frequencies to search genomes for disease-casing alleles and VAAST [2, 3] is a probabilistic disease-gene finder uses variant frequency and amino acid conservation information. To assemble these data, two copies of a known disease-causing allele randomly selected from HGMD [26] (see methods for details) can be inserted into a single target exome, repeating the process 100 times for 100 different known disease genes in order to determine margins of error. For these analyses, only SNVs can be used, excluding indels and other types of variants because not every variant prioritization tool can score them.

The heights of the bars in **FIG. 4A** summarize the percentage of the 100 trials in which the prioritization tool scored the known disease-causing allele. Importantly the percentages in **FIG. 4A** include all scored alleles, whether or not they are scored deleterious. For example SIFT scored 46% of the known disease-causing variants as either deleterious or tolerated. It may be unable to score the remaining 54% of the alleles. Annovar scored 95% of the alleles, and VAAST and PhastCons scored every allele. These percentages vary because not every tool is capable of scoring every potential disease-causing variant. The reasons vary from tool to tool, and case to case. SIFT, for example, cannot score alleles located in poorly conserved coding regions of genes [27].

The shadings of the bars in **FIG. 4** summarize the percentage of time the disease gene is ranked among the top ten candidates genome-wide (red), or among the top 100 candidates (blue), with white (color not labeled) denoting a rank greater than 100 in the candidate list. The table in **FIG. 4** summarizes this information in more detail. Annovar for example ranked 95% of the genes spiked with known disease-causing alleles as potentially damaged, judging the remainder of these genes as containing only non-deleterious alleles. Of the 95% of damaged genes it detected, on average it ranked all of them within the top 100 candidates genome-wide. For the 5% of genes that Annovar did not rank, a rank of 1,141 is assigned-the midpoint of the annotated 22,107 human genes; hence the average rank is much lower: 3,653. VAAST, by comparison, ranked every gene and identified the disease-causing gene among the top 100 candidates 99% of the time, with an average rank of 83 genome-wide. Note that in 100 runs of 100 different test cases, no tool ever places the disease-gene among the top 10 candidates. **FIG. 4A** thus illustrates a basic fact of personal genome analysis: using only a single affected exome, today's tools are underpowered to reliably identify the damaged gene and disease-causing variants.

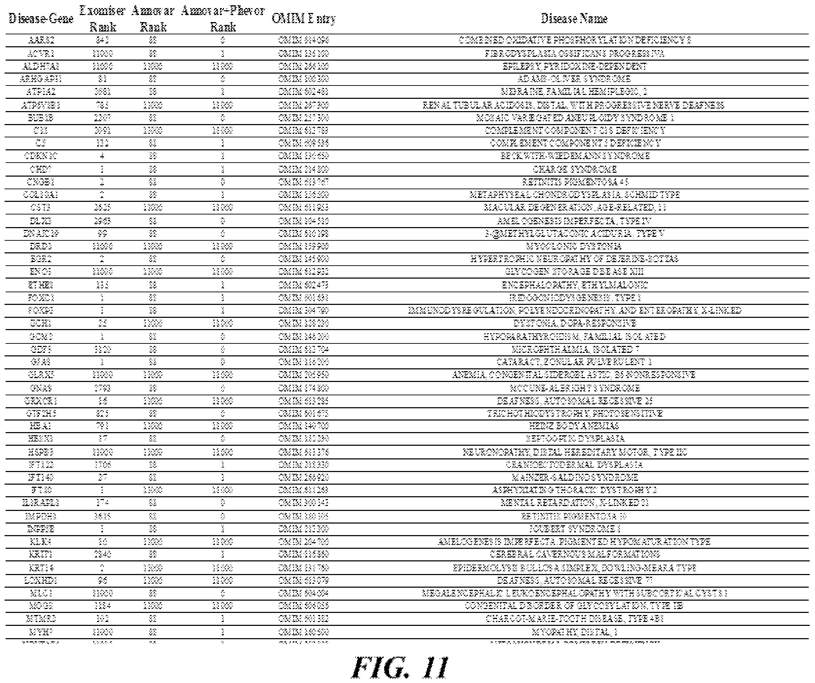

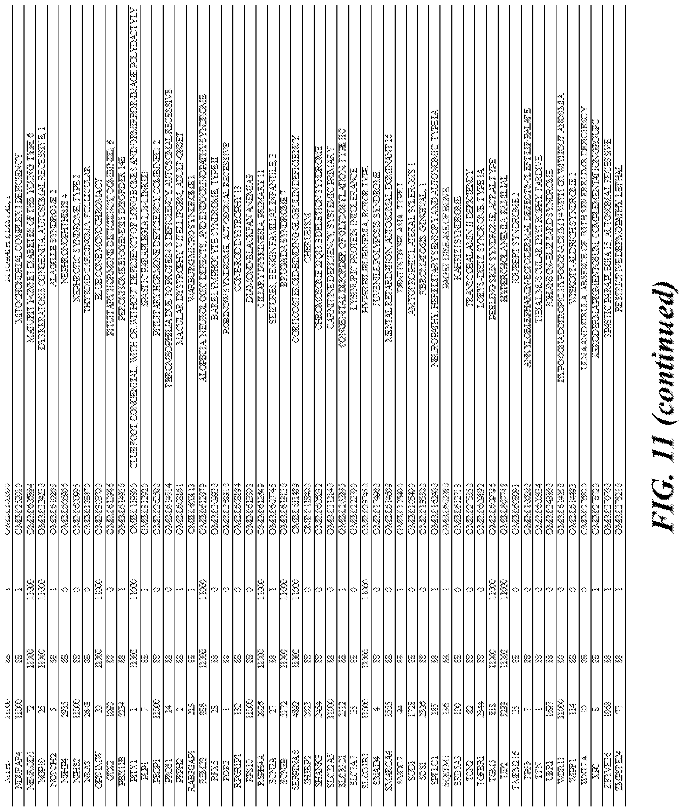

**FIG. 4B** summarizes the results of using Phevor to reanalyze the same SIFT, Annovar, PhastCons and VAAST output files used to produce **FIG. 4A****.** For these analyses, each tool's output files are provided to Phevor along with phenotype report containing the HPO terms annotated to each selected disease gene. These phenotype descriptions are provided in the table of **FIG. 11****.** As can be seen, Phevor dramatically improves the performance of each of the tools benchmarked in **FIG. 4A****.** For the 95% of genes ranked by Annovar, all are among the top 10 candidates, and Phevor improves the average rank for Annovar from 3,653 to 552. Similar trends are seen for SIFT. Even better improvements are seen with Phevor using PhastCons and VAAST outputs. The average rank for VAAST, for example, improves from 83 to 1.8, and 100% of the time the disease-gene is ranked in the top 10 genes. Phevor performs best on VAAST outputs because it has a lower false negative rate compared to SIFT and Annovar (**FIG. 4A**)**.** This is because Phevor improves the ranks of prioritized genes; it doesn't re-rank genes previously determined by a tool to harbor no deleterious alleles.

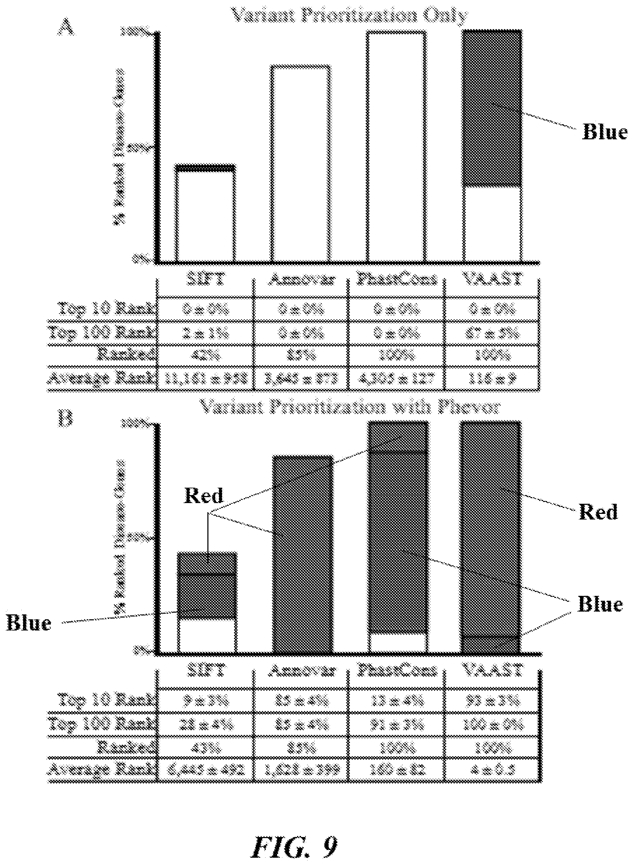

Results for dominant disease are provided in **FIG. 9. FIG. 9A** shows performance comparisons of four different variant prioritization tools before Phevor. **FIG. 9B** shows performance comparisons of four different variant prioritization tools after Phevor. A single copy of a known disease-causing allele is randomly selected from HGMD and spiked into a single target exome at the reported genomic location; hence these results model simple, dominant diseases. This process is repeated 100 times for 100 different, randomly selected known disease genes in order to determine margins of error. Bar charts show the percentage of time the disease gene is ranked among the top ten candidates genome-wide (red), or among the top 100 candidates (blue), with white (color not labeled) denoting a rank greater than 100 in the candidate list. For the Phevor analyses shown in the bottom panel, each tool's output files are fed to Phevor along with phenotype report containing the HPO terms annotated to each disease gene. The table below the bar charts summarizes this information in more detail. Bars do not reach 100% due to false negatives, i.e., the tool is unable to prioritize the disease-causing allele. Damaging alleles predicted to be benign are placed at the midpoint of the list 22,107 annotated human genes.

Benchmarks for dominant diseases show the same trends, with every tool exhibiting lower power relative to the recessive cases. However, Phevor still markedly improves power. Using VAAST, Phevor ranked the disease gene in the top 10 candidates 93% of the time.

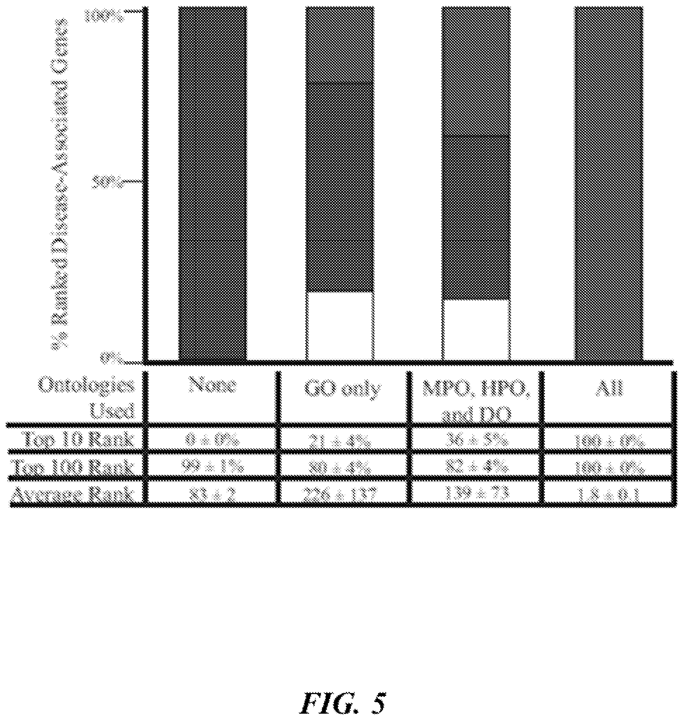

Collectively, these results demonstrate that Phevor can improve the power of widely used variant prioritization tools. Recall however, that the HPO provides a list of -2500 known human disease genes, each annotated to one or more HPO nodes, and that Phevor uses this information during the ontology combination propagation steps shown in **FIGs. 2** **and** **3****,** and described elsewhere herein. In light of this fact, the question naturally arises as to how dependent is Phevor upon the disease gene having been previously annotated to an ontology. **FIG. 5** addresses this issue.

**FIG. 5** illustrates variant prioritization for novel genes involved with known diseases. The procedure used to produce the bottom panel of **FIG. 4** is repeated, but this time the disease-gene's ontological annotations are removed from all but the specified ontologies prior to running Phevor. For purposes of economy, only VAAST results are shown. Removing all the disease-genes annotations from all ontologies mimics the case of a novel disease gene with unknown GO function, process and cellular location, never before associated with a known disease or phenotype. This is equivalent to running VAAST alone ('None'), and the leftmost bar chart and table column summarize these results. The right-hand bar and table column summarize the results of running VAAST + Phevor using current ontological annotations of the disease-genes ('ALL'). The 'GO only' column reports the results of removing the disease gene's phenotype annotations, depicting discovery success using only the GO ontological annotations. This column models the ability of Phevor to identify a novel disease gene when the gene is annotated to GO, but has no disease, human, or model-organism phenotype annotations. In contrast The 'MPO, HPO and DO' column assays the impact of removing a gene's GO annotations, but leaving its disease, human and model-organism phenotype annotations intact.

**FIG. 5** can employ the same procedure used to produce **FIG. 4****,** but with the disease-gene removed from one or more of the ontologies prior to running Phevor. This makes it possible to evaluate the ability of Phevor to improve the ranks of a disease gene in the absence of any ontological assignments (i.e., as if it are a novel disease gene, never before associated with a disease or phenotype). For these benchmarks, **FIG. 5** presents the results of experiments directed to assessing the impact of simultaneously masking the gene's HPO, MPO and DO phenotype annotations, and its GO annotations. Outputs using only VAAST outputs.

As can be seen, removing the gene from one or more ontologies does decrease Phevor's power to identify the gene, but does not eliminate it; demonstrating that Phevor is gaining power by combining multiple ontologies. Removing the target gene from GO, and using only the three phenotype ontologies (HPO, MPO, DO) the target disease gene is still ranked in the top 10 candidates 36% of the time, and among the top 100 candidates 82% of the time. By comparison, using VAAST alone the target gene is ranked among the top 10 and 100 candidates 0% and 99% of the time respectively. The 18% false negative rate is an artifact of the benchmark procedure and results from removing the gene from GO. Briefly, because the majority of human genes (18,824) are already annotated to GO, the prior expectation is that a novel disease gene is also more likely to be annotated to GO than not, causing Phevor to prefer candidates already annotated to GO in this benchmarking scenario.

Similar trends are seen using GO [14] alone. This time removing the gene for the MPO, HPO and DO, Phevor places the disease gene among the top ten candidates 21% of the time and among the top 100 candidates 80% of the time-still much better than using VAAST alone. Recall that for this analysis, Phevor is provided with only a phenotype description - not GO terms-and that the disease gene is removed from every ontology containing any phenotype data, e.g., the, HPO, the DO and the MPO. Thus, this increase in ranks (e.g., 21% vs. 0% in the top ten) is solely the result of Phevor's ability to integrate the Gene Ontology into a phenotype driven prioritization process, demonstrating that Phevor can use the GO to aid in discovery of new disease-genes and disease-causing alleles. Collectively, these results demonstrate that a significant portion of Phevor's power is derived from its ability to relate phenotype concepts in the HPO to gene function, process and location concepts modeled by the GO.

**FIG. 5** demonstrates that Phevor improves the performance of the variant prioritization tool for novel disease genes. This is possible because, even when a (novel) disease gene is absent in the HPO, Phevor can nonetheless assign it a high score for disease association (N_{g}) after information associated with its paralogs is propagated by Phevor from the HPO to GO. This is a complex point, and an illustration is helpful. Consider the case for two potassium transporters, A and B. Deleterious alleles in one (A) are known to cause cardiomyopathy, whereas gene B, as yet, has no disease associations. If gene A and B are both annotated in GO as potassium transporters, when Phevor propagates the HPO associations of Gene A to GO, the GO node potassium transporter will receive some score, which in turn will be propagated to gene B. Thus even though gene B is absent from the HPO, its Phevor disease association score will increase because of its GO annotation. This illustrates the simplest of cases. Many, more complex scenarios are possible. For example, gene A and B might be annotated to different nodes in GO, with gene B's disease association score being increased proportionally following propagation across GO. Importantly, neither of these scenarios is mutually exclusive.

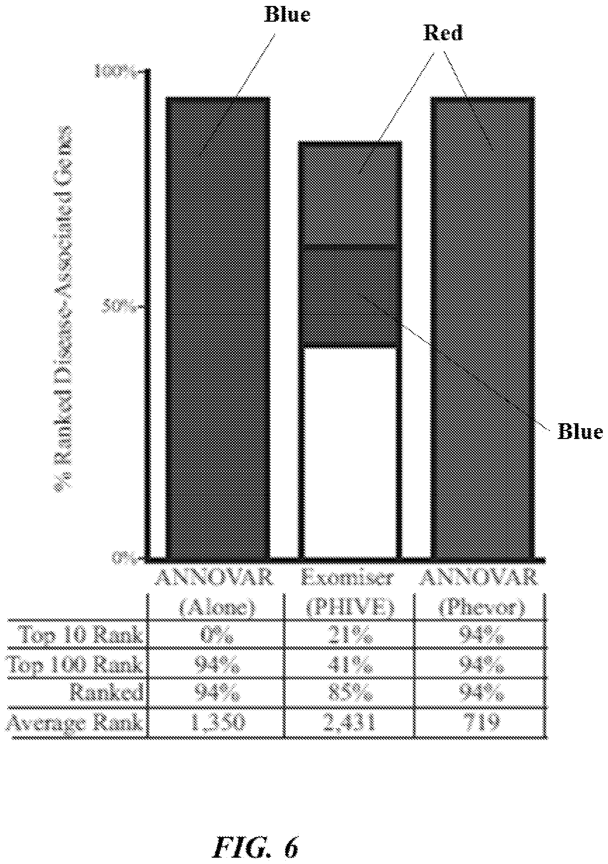

**FIG. 6** illustrates a comparison of Phevor to exomiser (PHIVE). This figure shows a comparison of disease-gene identification success rates for Phevor and the PHIVE methodology, which is available through the Exomiser web service. Exomiser is based upon Annovar's filtering logic, thus the Phevor comparison uses Annovar as the variant prioritization tool. The figure shows the results of 100 disease-gene searches of known recessive disease-genes. Identical variant files and phenotype descriptions are given to Exomiser+PHIVE and Annovar+Phevor. Bar charts show the percentage of time the disease gene is ranked among the top ten candidates genome-wide (red), or among the top 100 candidates (blue), with white (color not labeled) denoting a rank greater than 100 in the candidate list. The table below the bar charts summarizes this information in more detail. Bars do not reach 100% due to false negatives, i.e., the tool reported the disease-causing allele to be non-deleterious; these cases are placed at the midpoint of the list 22,107 annotated human genes.

The plots of **FIG. 6** are based on a comparison of the relative performance of Phevor to PHIVE [20], an online tool that uses Annovar in conjunction with human and mouse phenotype data to improve Annovar's prioritization accuracy. PHIVE is accessible through the Exomiser online tool [20]. For this benchmark, repeating the process used to produce **FIG. 4****,** two copies of a known disease-causing allele randomly selected from HGMD [26] (see methods for details) may be inserted into a target exome, repeating the process 100 different disease genes. The left-hand portion of **FIG. 6** provides a breakdown of the results when Annovar alone is used; the middle column reports the results of uploading these same 100 exomes to the Exomiser website; and the right column of **FIG. 6** shows the results for the same 100 exomes using Annovar with Phevor. As can be seen, the improvements in power by Phevor are considerable. Although Exomiser does increase the percentage of cases for which the target gene is located in the top ten and top 100 candidates compared to using Annovar alone, it does so at the expense of additional false negatives. In contrast Phevor obtains much better power on the same dataset (right-most plot of **FIG. 6****)** without incurring any additional false negatives. Phevor is, however, ultimately limited by Annovar's false negative rate. This limitation can be overcome simply by using VAAST reports instead of Annovar reports, in which case Phevor places 100% of the target genes among the top 10 candidates (c.f. **FIG. 4B****).**

The present disclosure also provides a determination of the impact of atypical disease presentation upon Phevor's accuracy. The term atypical presentation refers to cases in which an individual has a known genetic disease but does not present with the typical disease phenotype. Reasons include novel alleles in known disease genes, novel combinations of alleles, ethnicity (genetic background effects), environmental influences, and in some cases, multiple genetic diseases presenting in the same individual(s), to produce a compound phenotype [28]. Atypical presentation resulting from novel alleles in known disease genes and compound phenotypes due to disease-causing alleles are emerging as a common occurrence in personal genomes driven diagnosis [9, 29, 30]; thus, Phevor's performance in such situations is of interest.

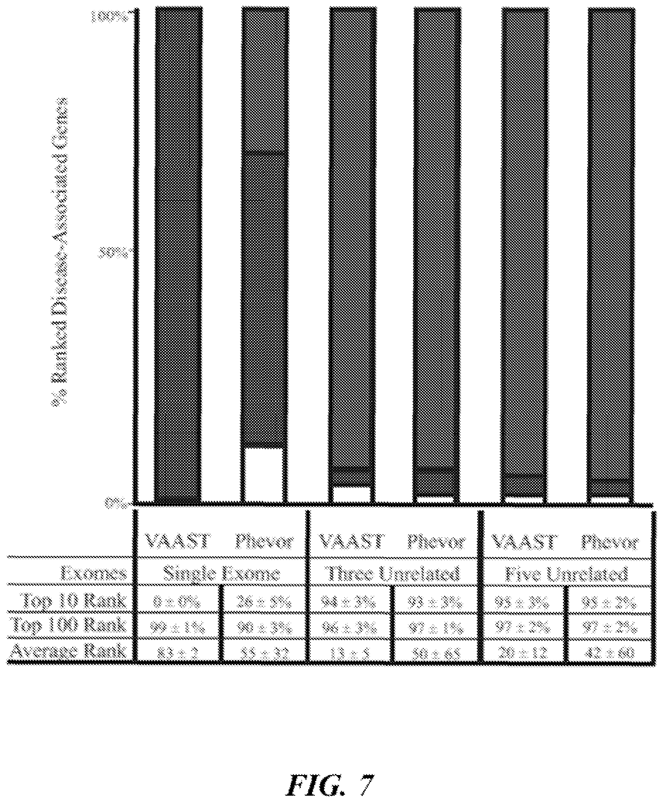

**FIG. 7** addresses the impact of atypical disease presentation on Phevor for case cohorts of 1, 3 and 5 unrelated individuals. In order to evaluate the impact of incorrect diagnosis or atypical phenotypic presentation on Phevor's accuracy, the analysis shown in **FIG. 4** can be repeated. The phenotype descriptions for each gene can be randomly shuffled at runtime, and the same phenotype descriptions for every member of a case cohort can be used. For reasons of economy, only VAAST results are shown. The results of running VAAST, with and without Phevor for 1, 3, and 5 unrelated individuals, are shown. Providing Phevor with incorrect phenotype data significantly impacts its diagnostic accuracy. For a single affected, power declines from the damaged gene being ranked in the top ten candidates genome-wide in 100% of the cases to 26% of cases. Nevertheless, Phevor is still able to improve upon VAAST's performance alone. Phevor places 95% of the disease genes in the top 10 candidates with cohorts of 3 and 5 unrelated affecteds, despite the misleading phenotype data, as the additional statistical power provided by VAAST increasingly outweighs the incorrect prior probabilities provided by Phevor.

With continued reference to **FIG. 7****,** each disease-gene's HPO-based phenotype description is randomly replaced with another's, thereby mimicking an extreme scenario of atypical presentation/mis-diagnosis, whereby each individual presents with not only an atypical phenotype, but still worse, one normally associated with some other known genetic disease. Unsurprisingly, this significantly impacts Phevor's' diagnostic accuracy. Using VAAST outputs, for a single affected individual, accuracy declines from the damaged gene being ranked in the top ten candidates genome-wide for 100% of the cases to 26%. More surprising is that Phevor is still able to improve on VAAST's performance alone, a phenomenon resulting again from Phevor's use of GO (as in **FIG. 6****).**

The remaining columns in **FIG. 7** measure the impact of increasing case cohort size. As can be seen, with 3 or more unrelated individuals all with the same (shuffled) atypical phenotypic presentation, Phevor performs very well, even when the phenotype information is misleading. Thus these results demonstrate how Phevor's ontology-derived scores, e.g., *N_{g}* in Equations 1 and 2, are gradually overridden in the face of increasing sequence-based experimental data to the contrary- a clearly desirable behavior.

The present disclosure also provides case studies in which Phevor is employed in tandem with Annovar and VAAST to identify disease-causing alleles in patients having an undiagnosed disease of likely genetic cause. All three cases involve small case cohorts containing related individuals or single affected exomes-scenarios for which existent prioritization tools are underpowered. These analyses thus demonstrate Phevor's utility using real clinical examples.

***NFKB2:* a new disease gene.** A family is identified to be affected by autosomal-dominant, early-onset hypogammaglobulinemia with variable autoimmune features and adrenal insufficiency. Blood samples are obtained from the affected mother and her two affected children, and from the unaffected father of the children (Family A). Blood is also obtained from a fourth, unrelated affected individual with the same phenotype (Family B). Sequencing is performed as described in [4], and variant annotation is performed using the VAAST Annotation Tool, VAT [3].

Exome data from the four individuals in Family A and the affected individual from Family B are then analyzed with VAAST [2, 3]. This analysis identified a deletion (c.2564delA) in the *NFKB2 gene* in Family A. This frameshift deletion changes the conserved Lys855 to a serine and introduces a premature stop codon at amino acid 861 of the NFKB2 gene. VAAST identified a second allele, also in NFKB2 in Family B, c.2557C>T; this mutation introduces a premature stop codon at amino acid 853. Subsequent immunoblot analysis and immunofluorescence microscopy of transformed B cells from affected individuals showed that the *NFKB2* mutations affect phosphorylation and proteasomal processing of the p100 NFKB2 protein to its p52 derivative and, ultimately, p52 nuclear translocation [4].

**FIG. 8A** shows the results of running Annovar (top left panel) and VAAST (top right panel) on the union of all variants identified in the affected children and their affected mother from Family A, combined with those of affected individual from Family B. The x-axes of the Manhattan plots in **FIG. 8A** are the genomic coordinates of the candidate genes. The y-axes show the log₁₀ value of the Annovar score, VAAST P-value, or Phevor score depending upon method. For proposes of comparison to VAAST, the Annovar scores may be transformed to frequencies, dividing the number of candidates by the total number of annotated human genes; hence there is a 'shelf of candidates in the Annovar plot at 1.14 on the y-axis. Both Annovar and VAAST identify a number of equally likely candidate genes. *NFKB2* (location marked for the Annovar panel only; the location in the other panels is the same as the Annoval panel) is among them in both analyses.

The lower panel of **FIG. 8A****,** presents the results of post-processing these same Annovar and VAAST outputs files using Phevor, together with a Phenomizer derived, HPO based phenotype description consisting of the following terms: Recurrent infections (HPO:0002719) and Abnormality of Humoral immunity (HPO:0005368). Phevor identifies a single best candidate, *NFKB2,* using the VAAST output, and the same gene ranks second using the Annovar output. Functional follow-up studies established *NFKB2,* and hence the non-canonical NF-κB signaling pathway, as a genetic etiology for this primary immunodeficiency syndrome [4]. Thus these analyses demonstrate PHEVOR's ability to identify a new human disease gene not currently associated with a disease or phenotype in the HPO, DO or MPO.

***STAT1:* An atypical phenotype caused by a known disease gene.** The proband is a 12-year-old male with severe diarrhea in the context of intestinal inflammation, total villous atrophy, and hypothyroidism. He required total parenteral nutrition to support growth, resulting in multiple hospitalizations for central line-associated bloodstream infections. During multidisciplinary comprehensive clinical evaluation, a diagnosis of IPEX syndrome (OMIM: 304790) may be considereed, but clinical sequencing of the *FOXP3* and *IL2RA* genes associated with IPEX [31, 32] may reveal no pathologic variants. His clinical picture is life threatening, warranting hematopoietic stem cell transplantation despite the diagnostic uncertainty. Prior to pre-transplant myeloablation, DNA is obtained from the proband and both parents. **FIG. 8B** shows the results of Annovar and VAAST analysis using the proband's exome. As is the case for *NFKB2,* both Annovar and VAAST are underpowered to distinguish the disease-gene and causative alleles from a background of other likely candidates. Phevor analyses of these same data, together with a phenotype description consisting of the HPO terms Hyopthryoidism (HP:0000812), Paronychia (HP:0001818), Autoimmunity (HP:0002960), and Abnormality of the intestine (HP:0002242) identified a single gene, *STAT1* as the 3^{rd}-ranked candidate in the Annovar outputs, and best candidate in the VAAST analyses (lower panels of **FIG. 8B**)**.**

Subsequent analyses of the proband's parents determined that the top scoring variant in the VAAST-Phevor run is a single *de novo* mutation in the DNA-binding region of *STAT1* (p.Thr3 85Met).

Multiple protein sequence alignment shows conservation across phyla at this amino acid position (data not shown). Moreover, gain-of-function mutations in *STAT1* cause immune mediated human disease [33] and *STAT1* is a transcription factor that regulates *FoxP3* [34]. Functional studies indicated that this mutation leads to an overexpression of STAT1 protein [34-36], suggesting gain-of-function mutation as a mechanism. Supporting this conclusion are the recent reports of this same allele causing chronic mucocutaneous candidiasis [37] and an IPEX-like syndrome [34]. These results highlight Phevor's ability, using only a single affected exome, to identify a mutation in a known human disease gene producing an atypical phenotype.

***ABCB11:* A new mutation in a known disease gene.** The Proband is a six-month old infant with an undiagnosed liver disease phenotypically similar to progressive familial intrahepatic cholestasis (PFIC) [38]. To identify mutations in the proband, exome sequencing is performed on the affected individual and both parents. Sequencing and bioinformatics processing are performed as described in the methods section.

For these Phevor analyses, a single HPO phenotype term is used: "intrahepatic cholestasis, HP:0001406". As shown in **FIG. 8C****,** Phevor analysis identified a single candidate gene (*ABCB11*) in the proband's exome sequence.

Mutations in *ABCB11* are known to cause progressive familial intrahepatic cholestasis Type 2. The variants identified by VAAST and supported as causative by Phevor form a compound heterozygote in the proband. These variants may be confirmed by Sanger sequencing, as described elsewhere herein. The paternal variant (chr2:169787254) causes a phenylalanine-to-serine amino acid substitution, while the maternal variant (chr2:169847329) produces a glutamic acid to glycine substitution. Both variants are considered highly damaging by SIFT. The maternal variant is known to cause intrahepatic cholestasis [39] while the paternal mutation is novel. These results demonstrate the utility of Phevor for identification of a new mutation in a known disease gene present *in trans* to a known allele and using only a single affected exome.

The present disclosure provides a series of benchmark and case studies demonstrating that Phevor can effectively improve the diagnostic power of widely used variant prioritization tools. These results demonstrate that Phevor is especially useful for single exome and small, family-based analyses, the most commonly occurring clinical scenarios, and ones for which existing variant prioritization tools are most inaccurate and underpowered.

Phevor's ability to improve the accuracy of variant prioritization tools may be the result of its ability to relate phenotype and disease concepts in ontologies such as HPO, and the DO to gene function, process and location concepts modeled by the GO. This allows Phevor to model key features of genetic disease that are not taken into account by existing methods [10, 20] that employ phenotype information for variant prioritization. For example, paralogous genes often produce similar diseases [40] because they have similar functions, operate in similar biological processes and are located in the same cellular compartments.

Phevor scores take into account not only weight of evidence that a gene is associated with the patient's illness, but that it is not. In typical whole exome searches every variant prioritization tool identifies many genes harboring what it considers to be deleterious mutations. Often the most damaging of them are found in genes without any known phenotype associating them with the disease of interest; moreover, in practice, highly deleterious alleles are also often false positive variant calls. Phevor successfully down weights these genes and alleles, with the target disease gene's rank climbing as an indirect result. This phenomenon is well illustrated by the fact that Phevor improves the accuracy of variant prioritization even when provided with an incorrect phenotype description, e.g., **FIG. 7****.** This result underscores the consistency of Phevor's approach; it also has some important implications. Namely, that lack of previous disease association, weak phylogenetic conservation, and lack of GO annotations for a gene are (weak) *prima facie* evidence against disease association.

The present disclosure also provides illustrations of the interplay of all of the above factors. Phevor can be employed in tandem with Annovar and VAAST to identify disease-causing alleles. In three example cases, small case cohorts containing either related individuals or single affected exomes are analyzed. For all these cases, variant prioritization alone is insufficient to identify the causative alleles, whereas when combined with Phevor, these same data revealed a single candidate. These analyses demonstrate Phevor's utility, using real clinical examples, to identify a novel recessive allele present as a compound heterozygote in a known disease gene (*ABCB11*); novel dominant alleles in a novel disease gene *(NFKB2);* and a *de novo* dominant allele in a known disease gene, resulting in an atypical phenotype (*STAT1*). Collectively these cases illustrate that Phevor can improve diagnostic accuracy for patients presenting with typical disease phenotypes, for patients with atypical disease presentations, and that Phevor can also use information latent in ontologies to discover new disease genes.

Phevor can provide researchers and healthcare professionals with an effective and improved approach to diagnose a genetic disease. As a first step in this direction, test datasets and a publically available Phevor web server can be used, which also provides the ability to enter, archive and update phenotype and variant data for use in sequence-based diagnosis. The Phevor web server can include a publically available web interface.

The incorporation of new ontologies gene-pathway information into Phevor is an active area of development. Phevor can employ any variant prioritization tool and any ontology-so long as it has gene annotations and is available in OBO format [41]. Over 50 biomedical ontologies, many satisfying both criteria, are publically available (e.g., The Open Biological and Biomedical Ontologies web site). Thus Phevor's approach should also prove useful for (non-) model organism and agricultural studies. Such applications raise interesting points. For the analyses presented here, the MPO may be used to leverage model organism phenotype data to improve diagnostic power for human patients. For model-, novel-organism, and agricultural applications, the HPO can be used in a manner analogous to that of the MPO in the analyses presented here, with Phevor systematically bringing human disease knowledge and human gene annotations to bear for non-model organism and agricultural studies.

Methods and systems of the present disclosure can be combined with or modified by other methods and systems, such as those described in Singleton, Marc V., et al. "Phevor Combines Multiple Biomedical Ontologies for Accurate Identification of Disease-Causing Alleles in Single Individuals and Small Nuclear Families," The American Journal of Human Genetics 94.4 (2014): 599-610 (including Supplemental Data), and U.S. Patent Publication Nos. 2007/0042369, 2012/0143512 and 2013/0332081; U.S. Patent No. 8,417,459; and PCT Publication Nos. WO/2004/092333 and WO/2012/034030, each of which is entirely incorporated herein by reference.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the invention described herein may be employed in practicing the invention. It is therefore contemplated that the invention shall also cover any such alternatives, modifications, variations or equivalents. It is intended that the following claims define the scope of the invention and that methods and structures within the scope of these claims and their equivalents be covered thereby.

### References:

1. Wang K, Li M, Hakonarson H: ANNOVAR: functional annotation of genetic variants from high-throughput sequencing data. Nucleic Acids Res 2010, 38:e164.
2. Hu H, Huff CD, Moore B, Flygare S, Reese MG, Yandell M: VAAST 2.0: Improved Variant Classification and Disease-Gene Identification Using a Conservation-Controlled Amino Acid Substitution Matrix. Genetic epidemiology 2013.
3. Yandell M, Huff C, Hu H, Singleton M, Moore B, Xing J, Jorde LB, Reese MG: A probabilistic disease-gene finder for personal genomes. Genome research 2011, 21:1529-1542.
4. Chen K, Coonrod EM, Kumanovics A, Franks ZF, Durtschi JD, Margraf RL, Wu W, Heikal NM, Augustine NH, Ridge PG, et al: Germline Mutations in NFKB2 Implicate the Noncanonical NF-kappaB Pathway in the Pathogenesis of Common Variable Immunodeficiency. Am J Hum Genet 2013.
5. Ng SB, Buckingham KJ, Lee C, Bigham AW, Tabor HK, Dent KM, Huff CD, Shannon PT, Jabs EW, Nickerson DA, et al: Exome sequencing identifies the cause of a mendelian disorder. Nature genetics 2010, 42:30-35.
6. Rope AF, Wang K, Evjenth R, Xing J, Johnston JJ, Swensen JJ, Johnson WE, Moore B, Huff CD, Bird LM, et al: Using VAAST to identify an X-linked disorder resulting in lethality in male infants due to N-terminal acetyltransferase deficiency. American journal of human genetics 2011, 89:28-43.
7. Shirley MD, Tang H, Gallione CJ, Baugher JD, Frelin LP, Cohen B, North PE, Marchuk DA, Comi AM, Pevsner J: Sturge-Weber syndrome and port-wine stains caused by somatic mutation in GNAQ. The New England journal of medicine 2013, 368:1971-1979.
8. McElroy JJ, Gutman CE, Shaffer CM, Busch TD, Puttonen H, Teramo K, Murray JC, Hallman M, Muglia LJ: Maternal coding variants in complement receptor 1 and spontaneous idiopathic preterm birth. Human genetics 2013, 132:935-942.
9. Yang Y, Muzny DM, Reid JG, Bainbridge MN, Willis A, Ward PA, Braxton A, Beuten J, Xia F, Niu Z, et al: Clinical whole-exome sequencing for the diagnosis of mendelian disorders. The New England journal of medicine 2013, 369:1502-1511.
10. Saunders CJ, Miller NA, Soden SE, Dinwiddie DL, Noll A, Alnadi NA, Andraws N, Patterson ML, Krivohlavek LA, Fellis J, et al: Rapid whole-genome sequencing for genetic disease diagnosis in neonatal intensive care units. Science translational medicine 2012, 4:154ra135.
11. Robinson PN, Kohler S, Bauer S, Seelow D, Horn D, Mundlos S: The Human Phenotype Ontology: a tool for annotating and analyzing human hereditary disease. American journal of human genetics 2008, 83:610-615.
12. Smith CL, Eppig JT: The Mammalian Phenotype Ontology as a unifying standard for experimental and high-throughput phenotyping data. Mammalian genome : official journal of the International Mammalian Genome Society 2012, 23:653-668.
13. Schriml LM, Arze C, Nadendla S, Chang YW, Mazaitis M, Felix V, Feng G, Kibbe WA: Disease Ontology: a backbone for disease semantic integration. Nucleic acids research 2012, 40:D940-946.
14. Ashburner M, Ball CA, Blake JA, Botstein D, Butler H, Cherry JM, Davis AP, Dolinski K, Dwight SS, Eppig JT, et al: Gene ontology: tool for the unification of biology. The Gene Ontology Consortium. Nature genetics 2000, 25:25-29.
15. Whetzel PL, Noy NF, Shah NH, Alexander PR, Nyulas C, Tudorache T, Musen MA: BioPortal: enhanced functionality via new Web services from the National Center for Biomedical Ontology to access and use ontologies in software applications. Nucleic acids research 2011, 39:W541-545.
16. Smith B, Ashburner M, Rosse C, Bard J, Bug W, Ceusters W, Goldberg LJ, Eilbeck K, Ireland A, Mungall CJ, et al: The OBO Foundry: coordinated evolution of ontologies to support biomedical data integration. Nature biotechnology 2007, 25:1251-1255.
17. Robinson PN, Bauer S: Introduction to bio-ontologies. Boca Raton: Taylor & Francis; 2011.
18. Ng PC, Henikoff S: Predicting the effects of amino acid substitutions on protein function. Annual review of genomics and human genetics 2006, 7:61-80.
19. Siepel A, Bejerano G, Pedersen JS, Hinrichs AS, Hou M, Rosenbloom K, Clawson H, Spieth J, Hillier LW, Richards S, et al: Evolutionarily conserved elements in vertebrate, insect, worm, and yeast genomes. Genome research 2005, 15:1034-1050.
20. Robinson P, Kohler S, Oellrich A, Wang K, Mungall C, Lewis SE, Washington N, Bauer S, Seelow DS, Krawitz P, et al: Improved exome prioritization of disease genes through cross species phenotype comparison. Genome research 2013.
21. Kohler S, Bauer S, Mungall CJ, Carletti G, Smith CL, Schofield P, Gkoutos GV, Robinson PN: Improving ontologies by automatic reasoning and evaluation of logical definitions. BMC Bioinformatics 2011, 12:418.
22. Online Mendelian Inheritance in Man, OMIM (TM). McKusick-Nathans Institute of Genetic Medicine, Johns Hopkins University (Baltimore, MD) and National Center for Biotechnology Information, National Library of Medicine (Bethesda, MD).
23. McKenna A, Hanna M, Banks E, Sivachenko A, Cibulskis K, Kernytsky A, Garimella K, Altshuler D, Gabriel S, Daly M, DePristo MA: The Genome Analysis Toolkit: a MapReduce framework for analyzing next-generation DNA sequencing data. Genome research 2010, 20:1297-1303.
24. Consortium TGP: A map of human genome variation from population-scale sequencing. Nature 2010, 467:1061-1073.
25. **VCF (Variant Call Format) version 4.0** [http://www.1000genomes.org/wiki/Analysis/vef4.0]
26. Cooper DN, Ball EV, Krawczak M: The human gene mutation database. Nucleic Acids Res 1998, 26:285-287.
27. Kumar P, Henikoff S, Ng PC: Predicting the effects of coding non-synonymous variants on protein function using the SIFT algorithm. Nature protocols 2009, 4:1073-1081.
28. Roach J, Glusman G, Smit A, Huff C, Hubley R, Shannon P, Rowen L, Pant K, Goodman N, Bamshad M, et al: Analysis of genetic inheritance in a family quartet by whole-genome sequencing. Science 2010, 328:636-639.
29. Roach JC, Glusman G, Smit AF, Huff CD, Hubley R, Shannon PT, Rowen L, Pant KP, Goodman N, Bamshad M, et al: Analysis of genetic inheritance in a family quartet by whole-genome sequencing. Science 2010, 328:636-639.
30. Boycott KM, Vanstone MR, Bulman DE, MacKenzie AE: Rare-disease genetics in the era of next-generation sequencing: discovery to translation. Nature reviews Genetics 2013, 14:681-691.
31. Bennett CL, Christie J, Ramsdell F, Brunkow ME, Ferguson PJ, Whitesell L, Kelly TE, Saulsbury FT, Chance PF, Ochs HD: The immune dysregulation, polyendocrinopathy, enteropathy, X-linked syndrome (IPEX) is caused by mutations of FOXP3. Nature genetics 2001, 27:20-21.
32. Caudy AA, Reddy ST, Chatila T, Atkinson JP, Verbsky JW: CD25 deficiency causes an immune dysregulation, polyendocrinopathy, enteropathy, X-linked-like syndrome, and defective IL-10 expression from CD4 lymphocytes. The Journal of allergy and clinical immunology 2007, 119:482-487.
33. Boisson-Dupuis S, Kong XF, Okada S, Cypowyj S, Puel A, Abel L, Casanova JL: Inborn errors of human STAT1: allelic heterogeneity governs the diversity of immunological and infectious phenotypes. Current opinion in immunology 2012, 24:364-378.
34. Uzel G, Sampaio EP, Lawrence MG, Hsu AP, Hackett M, Dorsey MJ, Noel RJ, Verbsky JW, Freeman AF, Janssen E, et al: Dominant gain-of-function STAT1 mutations in FOXP3 wild-type immune dysregulation-polyendocrinopathy-enteropathy-X-linked-like syndrome. The Journal of allergy and clinical immunology 2013, 131:1611-1623.
35. Sampaio EP, Hsu AP, Pechacek J, Bax HI, Dias DL, Paulson ML, Chandrasekaran P, Rosen LB, Carvalho DS, Ding L, et al: Signal transducer and activator of transcription 1 (STAT1) gain-of-function mutations and disseminated coccidioidomycosis and histoplasmosis. The Journal of allergy and clinical immunology 2013, 131:1624-1634.
36. Takezaki S, Yamada M, Kato M, Park MJ, Maruyama K, Yamazaki Y, Chida N, Ohara O, Kobayashi I, Ariga T: Chronic mucocutaneous candidiasis caused by a gain-of-function mutation in the STAT1 DNA-binding domain. Journal of immunology 2012, 189:1521-1526.
37. van de Veerdonk FL, Plantinga TS, Hoischen A, Smeekens SP, Joosten LA, Gilissen C, Arts P, Rosentul DC, Carmichael AJ, Smits-van der Graaf CA, et al: STAT1 mutations in autosomal dominant chronic mucocutaneous candidiasis. The New England journal of medicine 2011, 365:54-61.
38. Baghdasaryan A, Chiba P, Trauner M: Clinical application of transcriptional activators of bile salt transporters. Molecular aspects of medicine 2013.
39. Strautnieks SS, Bull LN, Knisely AS, Kocoshis SA, Dahl N, Arnell H, Sokal E, Dahan K, Childs S, Ling V, et al: A gene encoding a liver-specific ABC transporter is mutated in progressive familial intrahepatic cholestasis. Nature genetics 1998, 20:233-238.
40. Yandell M, Moore B, Salas F, Mungall C, MacBride A, White C, Reese MG: Genome-wide analysis of human disease alleles reveals that their locations are correlated in paralogous proteins. PLoS computational biology 2008, 4:e1000218.
41. **The OBO Flat File Format Specification, version 1.2** [http://www.geneontology.org/GO.format.obo-1_2.shtml]

Aspects of the invention are described below:
1. A computer system for identifying phenotype causing genetic variants, comprising:
   computer memory having a plurality of phenotype causing genes or genetic variants; and
   a computer processor coupled to said computer memory and said database, wherein said computer processor is programmed to (i) identify a first set of phenotype causing genes or genetic variants, which first set of phenotype causing genes or genetic variants is among said plurality of phenotype causing genes or genetic variants in said computer memory; (ii) prioritize said first set of phenotype causing genes or genetic variants based on knowledge resident in one or more biomedical ontologies in a database; (iii) automatically identify and report a second set of phenotype causing genes or genetic variants, wherein a priority ranking associated with genes or genetic variants in said second set of genes and genetic variants is improved compared to a priority ranking associated with said first set of phenotype causing genes or genetic variants.
2. The system of aspect 1, wherein said database is separate from said computer system.
3. The system of aspect 1, further comprising a communication interface for obtaining genetic information of a subject.
4. The system of aspect 3, wherein said computer processor is further programmed to use said second set of phenotype causing genes or genetic variants to analyze said genetic information of said subject to identify a phenotype or disease condition in said subject.
5. The system of aspect 4, wherein said computer processor is further programmed to generate a report that indicates said phenotype or disease condition in said subject.
6. The system of aspect 4, wherein said computer processor is further programmed to generate a report includes a diagnosis of a disease in said subject and/or recommends a therapeutic intervention for said subject.
7. The system of aspect 5 or 6, wherein said report is provided for display on a user interface on an electronic display.
8. The system of aspect 1, wherein said computer processor is further programmed to provide said second set of phenotype causing genes or genetic variants on a user interface.
9. A method for identifying phenotype-causing genetic variants, comprising:
   (a) providing a computer processor coupled to computer memory that includes a plurality of phenotype causing genes or genetic variants, wherein said computer processor is programmed to identify and prioritize sets of phenotype causing genes or genetic variants among said plurality of phenotype causing genes or genetic variants;
   (b) using said computer processor to identify a first set of phenotype causing genes or genetic variants, which first set of phenotype causing genes or genetic variants is among said plurality of phenotype causing genes or genetic variants in said computer memory;
   (c) prioritizing said first set of phenotype causing genes or genetic variants based on knowledge resident in one or more biomedical ontologies; and
   (d) automatically identifying and reporting on a user interface a second set of phenotype causing genes or genetic variants, wherein a priority ranking associated with genes or genetic variants in said second set of genes and genetic variants is improved compared to a priority ranking associated with said first set of phenotype causing genes or genetic variants.
10. The method of aspect 9, further comprising using said programmed computer processor to integrate personal genomic data, gene function, and disease information with phenotype or disease description of an individual for improved accuracy to identify phenotype-causing variants or genes (Phevor).
11. The method of aspect 9 or 10, further comprising using an algorithm that propagates information across and between ontologies.
12. The method of aspect 9 or 10, further comprising accurately reprioritizing damaging genes or genetic variants identified in said first set of genes or genetic variants based on gene function, disease and phenotype knowledge.
13. The method of aspect 9 or 10, further comprising incorporating a genomic profile of a single individual, wherein said genetic profile comprises single nucleotide polymorphisms, set of one or more genes, an exome or a genome, a genomic profile of one or more individuals analyzed together, or genomic profiles from individuals from a family.
14. The method of aspect 9 or 10, wherein said method improves diagnostic accuracy for individuals presenting with established disease phenotypes.
15. The method of aspect 9 or 10, wherein said method improves diagnostic accuracy for patients with novel or atypical disease presentations.
16. The method of aspect 9 or 10, further comprising incorporating latent information in ontologies to discover new disease genes or disease causing-alleles.
17. The method of aspect 9 or 10, wherein said first set of phenotype causing genes or genetic variants is identified by:
   using said computer processor to prioritize genetic variants by combining (1) variant prioritization information, (2) said knowledge resident in said one or more biomedical ontologies, and (3) a summing procedure; and
   automatically identifying and reporting said phenotype causing genes or genetic variants.
18. The method of aspect 17, wherein a phenotype description of sequenced individual(s) is included in said summing procedure.
19. The method of aspect 18, wherein said variant prioritization information is at least partially based on sequence characteristics selected from the group consisting of an amino acid substitution (AAS), a splice site, a promoters, a protein binding site, an enhancer, and a repressor.
20. The method of aspect 18, wherein said variant prioritization information is at least partially based on methods selected from the group consisting of VAAST, pVAAST, SIFT, ANNOVAR, burden-tests, and sequence conservation tools.
21. The method of aspect 18, wherein said one or more biomedical ontologies includes one or more of the Gene Ontology, Human Phenotype Ontology and Mammalian Phenotype Ontology.
22. The method of aspect 18, wherein said summing procedure comprises traversal of said ontologies, propagation of information across said ontologies and combination of one or more results of transversal and propagation, to produce a gene score which embodies a prior-likelihood that a given gene has an association with a user described phenotype or gene function.
23. The method of aspect 18, wherein said variant prioritization information is performed using a variant protein impact score and/or frequency information.
24. The method of aspect 23, wherein said impact score is selected from the group consisting of SIFT, Polyphen, GERP, CADD, PhastCons and PhyloP.
25. The method of aspect 18, wherein said phenotype description of said sequenced individual(s) is derived from a physical examination by a healthcare professional.
26. The method of aspect 18, wherein said phenotype description of said sequenced individual(s) is stored in an electronic medical health record.
27. The method of aspect 18, wherein said variants are prioritized in a genomic region comprising one or more genes or gene fragments, one or more chromosomes or chromosome fragments, one or more exons or exon fragments, one or more introns or intron fragments, one or more regulatory sequences or regulatory sequence fragments, or a combination thereof.
28. The method of aspect 18, wherein said biomedical ontologies are gene ontologies containing information with respect to gene function, process and location, disease ontologies containing information about human disease; phenotype ontologies containing knowledge concerning mutation phenotypes in non-human organisms, and information pertaining to paralogous and homologues genes and their mutant phenotypes in humans and other organisms.
29. The method of aspect 18, wherein said sequenced individuals are of different species.
30. The method of aspect 18, wherein said phenotype is a disease.
31. The method of aspect 18, wherein family phenotype information on affected and non-affected individuals is included in said phenotype description.
32. The method of aspect 18, further comprising including set(s) of family genomic sequences.
33. The method of aspect 32, further comprising incorporating a known inheritance mode.
34. The method of aspect 17 or 18, further comprising including sets of affected and non-affected genomic sequences.
35. The method of aspect 17 or 18, wherein said summing procedure is ontological propagation, and wherein seed nodes in some ontology are identified, each seed node is assigned a value greater than zero, and this information is propagated across said ontology.
36. The method of aspect 35, further comprising proceeding from each seed node toward its children nodes, wherein when an edge to a neighboring node is traversed, a current value of a previous node is divided by a constant value.
37. The method of aspect 36, wherein said summing procedure is that upon completion of propagation , each node's value is renormalized to a value between zero and one by dividing by a sum of all nodes in said ontology.
38. The method of aspect 37, wherein:
   (i) each gene annotated to an ontology receives a score corresponding to a maximum score of any node in said ontology to which that gene is annotated; and
   (ii) repeating (i) for each ontology, wherein genes annotated to a plurality of ontologies have a score from each ontology, and wherein scores from said plurality of ontologies are aggregated to produce a final sum score for each gene, and renormalized again to a value between one and zero.
39. The method of aspect 18, wherein said sequenced individual(s) have genetic sequences that are from one or more cancer tissue and germline tissue.
40. The method of aspect 18, further comprising:
   (i) scoring both coding and non-coding variants; and
   (ii) evaluating a cumulative impact of both types of variants in the context of gene scores, wherein (1) said variants are prioritized in a genomic region comprising one or more genes or gene fragments, one or more chromosomes or chromosome fragments, one or more exons or exon fragments, one or more introns or intron fragments, one or more regulatory sequences or regulatory sequence fragments, or a combination thereof, and/or (2) said biomedical ontologies are gene ontologies containing information with respect to gene function, process and location, disease ontologies containing information about human disease; phenotype ontologies containing knowledge concerning mutation phenotypes in non-human organisms, and information pertaining to paralogous and homologues genes and their mutant phenotypes in humans and other organisms.
41. The method of aspect 18, further comprising incorporating both rare and common variants to identify variants responsible for common phenotypes.
42. The method of aspect 41, wherein said common phenotypes include a common disease.
43. The method of aspect 18, further comprising identifying rare variants causing rare phenotypes.
44. The method of aspect 43, wherein said rare phenotypes include a rare disease.
45. The method of aspect 9, wherein said knowledge includes phenogenomic information.
46. The method of aspect 9, 10 or 17, wherein said method has a statistical power at least 10 times greater than a statistical power of a method not using knowledge resident in one or more biomedical ontologies.
47. The method of aspect 9, 10 or 17, further comprising assessing a cumulative impact of variants in both coding and non-coding regions of a genome.
48. The method of aspect 9, 10 or 17, further comprising analyzing low-complexity and repetitive genome sequences.
49. The method of aspect 9, 10 or 17, further comprising analyzing pedigree data.
50. The method of aspect 9, 10 or 17, further comprising analyzing phased genome data.
51. The method of aspect 9, 10 or 17, wherein family information on affected and non-affected individuals is included in a target and background database.
52. The method of aspect 9, 10 or 17, used in conjunction with a method for calculating a composite likelihood ratio (CLR) to evaluate whether a genomic feature contributes to a phenotype.
53. The method of aspect 9, 10 or 17, further comprising calculating a disease association score (D_{g}) for each gene, wherein D_{g} = (1-V_{g}) x N_{g}, wherein N_{g} is a renormalized gene sum score derived from ontological propagation, and V_{g} is a percentile rank of a gene provided by said variant prioritization tool.
54. The method of aspect 53, further comprising calculating a healthy association score (H_{g}) summarizing a weight of evidence that a gene is not involved with an illness of an individual, wherein, H_{g} = V_{g} x (1-N_{g}).
55. The method of aspect 54, further comprising calculating a final score (S_{g}) as a log₁₀ ratio of disease association score (D_{g}) and said healthy association score (H_{g}), wherein S_{g} = log₁₀ D_{g}/H_{g}.
56. The method of aspect 55, further comprising using a magnitude of Sg to re-rank each gene in said second set of phenotype causing genes or genetic variants.
57. The method of aspect 9, 10 or 17, wherein said user interface is a graphical user interface (GUI) of an electronic device of a user, which GUI has one or more graphical elements selected to display said second set of phenotype causing genes or genetic variants.
58. The method of aspect 9, 10 or 17, wherein said first and/or second set of phenotype causing genes or genetic variants are genetic markers.
59. The method of aspect 9, 10 or 17, wherein said first set of phenotype causing genes or genetic variants is associated with a first set of ranking scores, said second set of phenotype causing genes or genetic variants is associated with a second set of ranking scores, wherein said second set of ranking scores is improved with respect to said first set of ranking scores.
60. The method of aspect 9, 10 or 17, further comprising obtaining genetic information of a subject, and using said second set of phenotype causing genes or genetic variants to analyze said genetic information of said subject to identify a phenotype or disease condition in said subject.
61. The method of aspect 60, wherein the genetic information of said subject is obtained by sequencing, array hybridization or nucleic acid amplification using markers that are selected to identify said phenotype causing genes or genetic variants of said second set.
62. The method of aspect 60, further comprising diagnosing a disease of said subject and/or recommending a therapeutic intervention for said subject.
63. The method of aspect 17, wherein said variant prioritization information of said first set of phenotype causing genes or genetic variants comprises use of family genomic sequences of affected or non-affected family members.
64. The method of aspect 63, wherein use of family genomic sequences comprises incorporating an inheritance mode based one or more of autosomal recessive, autosomal dominant, and x-lined.
65. The method of aspect 9, further comprising prioritizing and identifying disease causing genetic markers from a third set of phenotype causing genes or genetic variants based on said knowledge.
66. The method of aspect 9, further comprising incorporating genomic profiles of one or more individuals, wherein said genomic profiles comprise measurements of one or more of the following: one or more single nucleotide polymorphisms, one or more genes, one or more exomes, and one or more genomes.
67. The method of aspect 9, wherein a statistical power generated by said prioritizing analysis based on a combination of said one or more biomedical ontologies and genomic data is at least 10 times greater than a statistical power generated by said prioritizing analysis based on said one or more biomedical ontologies or said genomic data, but not both.
68. The method of aspect 9, further comprising integrating said knowledge resident in one or more biomedical ontologies with an individual's phenotype or disease description to identify a third set of phenotype causing genes or genetic variants from said first and/or second sets of phenotype causing genes or genetic variants.
69. The method of aspect 68, wherein said third set of phenotype causing genes or genetic variants recognizes phenotype(s) with an improved accuracy measure with respect to said first and second sets of phenotype causing genes or genetic variants.
70. The method of Aspect 17 or 18, wherein said summing procedure is ontological propagation, and wherein one or more seed nodes are identified using one or more phenotype descriptions for a subject.
71. The method of Aspect 70, wherein said one or more seed nodes are identified using a plurality of phenotype descriptions.
72. The method of Aspect 70, further comprising repeating (b)-(d) at least once using one or more different phenotype descriptions to yield an improved priority ranking.
73. A method for identifying phenotype-causing genetic variants, comprising:
   (a) providing a computer processor coupled to computer memory that includes a plurality of phenotype causing genes or genetic variants, wherein said computer processor is programmed to identify and prioritize sets of phenotype causing genes or genetic variants among said plurality of phenotype causing genes or genetic variants;
   (b) using said computer processor to identify a first set of phenotype causing genes or genetic variants, which first set of phenotype causing genes or genetic variants is among said plurality of phenotype causing genes or genetic variants in said computer memory;
   (c) prioritizing said first set of phenotype causing genes or genetic variants based on knowledge resident in one or more biomedical ontologies;
   (d) automatically identifying a second set of phenotype causing genes or genetic variants, wherein a priority ranking associated with genes or genetic variants in said second set of genes and genetic variants is improved compared to a priority ranking associated with said first set of phenotype causing genes or genetic variants; and
   (e) using said second set of phenotype causing genes or genetic variants to analyze genetic information of a subject to identify a phenotype or disease condition in said subj ect.
74. The method of aspect 73, further comprising using said programmed computer processor to integrate personal genomic data, gene function, and disease information with phenotype or disease description of an individual for improved accuracy to identify phenotype-causing variants or genes (Phevor).
75. The method of aspect 74, wherein said first set of phenotype causing genes or genetic variants is identified by:
   using said computer processor to prioritize genetic variants by combining (1) variant prioritization information, (2) said knowledge resident in said one or more biomedical ontologies, and (3) a summing procedure; and
   automatically identifying and reporting said phenotype causing genes or genetic variants.
76. The method of aspect 73, further comprising obtaining said genetic information of said subj ect.
77. The method of aspect 76, wherein the genetic information of said subject is obtained by sequencing, array hybridization or nucleic acid amplification using markers that are selected to identify said phenotype causing genes or genetic variants of said second set.
78. The method of aspect 73, 74 or 75, further comprising diagnosing a disease of said subject and/or recommending a therapeutic intervention for said subject.

## Claims

1. A computer-implemented method for identifying phenotype-causing genetic variants, comprising:
(a) providing to a computer processor coupled to computer memory a plurality of phenotype-causing genes or genetic variants;
(b) using said computer processor to identify and prioritize sets of phenotype-causing genes or genetic variants among said plurality of phenotype-causing genes or genetic variants;
(c) using said computer processor to identify a first set of phenotype-causing genes or genetic variants, wherein said first set of phenotype-causing genes or genetic variants is among said plurality of phenotype-causing genes or genetic variants;
(d) prioritizing said first set of phenotype-causing genes or genetic variants based on knowledge resident in a set of gene ontologies;
(e) automatically identifying a second set of phenotype-causing genes or genetic variants, wherein a priority ranking associated with phenotype-causing genes or genetic variants in said second set of phenotype-causing genes and genetic variants is improved compared to a priority ranking associated with said first set of phenotype-causing genes or genetic variants; and
(f) using said second set of phenotype-causing genes or genetic variants to analyze genetic information of an individual to automatically identify and report on a user interface a phenotype or disease condition in said individual.

2. The computer-implemented method of claim 1, further comprising integrating personal genomic data, gene function, and disease information with phenotype or disease description of an individual for improved accuracy to identify phenotype-causing genes or genetic variants.

3. The computer-implemented method of claim 2, wherein said first set of phenotype-causing genes or genetic variants is identified by:
using said computer processor to prioritize genes or genetic variants by combining (1) variant prioritization information, (2) said knowledge resident in said one or more biomedical ontologies, and (3) a summing procedure; and
automatically identifying and reporting on said user interface said phenotype-causing genes or genetic variants.

4. The computer-implemented method of claim 1, further comprising obtaining said genetic information of said individual.

5. The computer-implemented method of claim 4, wherein said genetic information of said individual is obtained by sequencing, array hybridization or nucleic acid amplification using markers that are selected to identify said phenotype-causing genes or genetic variants of said second set.

6. The computer-implemented method of any one of claims 1-5, further comprising diagnosing a disease of said individual.

7. The computer-implemented method of any one of claims 1-6, further comprising recommending a therapeutic intervention for said individual.

8. The computer-implemented method of claim 1, wherein said plurality of phenotype-causing genes or genetic variants comprises disease-causing genes or genetic variants.

9. The computer-implemented method of claim 1, wherein (e) comprises identifying said disease condition in said individual.

10. The computer-implemented method of claim 1, wherein said set of gene ontologies is represented as a directed acyclic graph, wherein disease phenotypes are nodes in said directed acyclic graph, and any genes associated to said nodes are assigned a value.

11. The computer-implemented method of claim 1, wherein said plurality of phenotype-causing genes or genetic variants are scored based on a characteristic comprising a sequence characteristic selected from the group consisting of an amino acid substitution (AAS), a splice site, a promoter, a protein binding site, an enhancer, and a repressor.

12. The computer-implemented method of claim 1, wherein said plurality of phenotype-causing genes or genetic variants have been previously ranked by VAAST, pVAAST, SIFT, ANNOVAR, a burden-test, a sequence conservation scoring method, or a functional gene or variant sequence scoring method.

13. The computer-implemented method of claim 1, wherein said set of gene ontologies contain knowledge concerning mutation phenotypes in non-human organisms, and information pertaining to paralogous and homologues genes and their mutant phenotypes in humans and other organisms.

14. The computer-implemented method of claim 1, further comprising prioritizing damaging genes or genetic variants in said first set of phenotype-causing genes or genetic variants based on gene function, disease and phenotype knowledge.

15. The computer-implemented method of claim 1, wherein said set of gene ontologies comprise Human Phenotype Ontology (HPO).
